# EUROPÄISCHE PATENTSCHRIFT

(11) **EP 4 452 043 B1**
(45) Veröffentlichungstag und Bekanntmachung des Hinweises auf die Patenterteilung: **16.09.2026**
(21) Anmeldenummer: 22840154.3
(22) Anmeldetag: 19.12.2022
(51) Int. Cl.: A61B 3/00, G02B 19/00, G02B 27/09, G02C 13/00

(54) **FIXATIONSTARGET, ZENTRIERVORRICHTUNG UND VERWENDUNG**
FIXATION TARGET, CENTRATION APPARATUS AND USE
CIBLE DE FIXATION, APPAREIL DE CENTRAGE ET UTILISATION

(30) Priorität: 23.12.2021 DE 102021214978
(43) Veröffentlichungstag der Anmeldung: 30.10.2024
(73) Patentinhaber: Rodenstock GmbH, 80687 München (DE)
(72) Erfinder: STUTZ, Michel, 81541 München (DE); GROMANN, Lukas, 85354 Freising (DE); TRUMM, Stephan, 80999 München (DE)
(74) Vertreter: Müller-Boré & Partner Patentanwälte PartG mbB
(86) Internationale Anmeldenummer: PCT/EP2022/086760
(87) Internationale Veröffentlichungsnummer: WO 2023/117978

(56) Entgegenhaltungen:
- EP-B1- 2 056 067
- CN-C- 100 412 646
- DE-A1- 4 320 177
- DE-B4- 102008 003 906
- JP-A- 2008 275 559
- US-A- 5 568 313
- US-A1- 2011 236 651
- GRINTECH GMBH: "Gradient Index (GRIN) Lenses", 1 December 2015 (2015-12-01), XP055830340, Retrieved from the Internet <URL:https://www.grintech.de/fileadmin/user_upload/Introduction_GRIN_Lenses.pdf> [retrieved on 20210805]

## Beschreibung

Die Erfindung betrifft ein Fixationstarget, eine Zentriervorrichtung, eine Verwendung eines Fixationstargets und ein Verfahren.

### STAND DER TECHNIK

Durch die Einführung von individuell optimierten Brillengläsern ist es möglich, auf die Ansprüche von Personen mit Sehfehlern einzugehen und beispielsweise Brillengläser mit individuell optimierten Sehbereichen bereitzustellen. Individuell angepasste Brillengläser ermöglichen eine optimale Korrektur von optischen Sehfehlern eines Benutzers der Brillengläser. Eine individuelle Berechnung und Anpassung von Brillengläsern ist auch für Sportbrillen möglich, welche sich durch große Durchbiegungen, Fassungsscheiben- und Vorneigungswinkel auszeichnen.

Um die optischen Vorteile von individuellen Brillengläsern, insbesondere von individuell angepassten Gleitsichtgläsern, vollständig auszuschöpfen, ist es notwendig, diese Brillengläser in Kenntnis der Gebrauchsstellung des Benutzers zu berechnen und herzustellen und gemäß der zur Berechnung und Herstellung verwendeten Gebrauchsstellung zu tragen. Die Gebrauchsstellung ist von einer Vielzahl von optischen Zentrierparametern abhängig, beispielsweise von der Pupillendistanz des Benutzers, dem Fassungsscheibenwinkel, der Brillenglasvorneigung, der Brillenfassung, dem Hornhautscheitelabstand des Systems von Brille und Auge und der Einschleifhöhe der Brillengläser. Diese und weitere Parameter, welche zur Beschreibung der Gebrauchsstellung herangezogen werden können, bzw. notwendig sind, sind in einschlägigen Normen, wie beispielsweise der DIN EN ISO 13666, der DIN 58 208, der DIN EN ISO 8624 und der DIN 5340 enthalten und können diesen entnommen werden.

Dabei können die Brillengläser entsprechend den optischen Zentrierparametern, welche zur Herstellung verwendet wurden, in einer Brillenfassung angeordnet bzw. zentriert werden, so dass die Brillengläser vom Brillenträger tatsächlich entsprechend den optischen Zentrierparametern in Gebrauchsstellung getragen werden.

Um die einzelnen optischen Zentrierparameter zu bestimmen, stehen dem Optiker eine Vielzahl von Messgeräten zur Verfügung, insbesondere Zentriervorrichtungen. Eine solche Zentriervorrichtung ist z.B. aus der DE 10 2005 003 699 A1 bekannt. Hierbei werden aus zumindest zwei Bildaufnahmerichtungen Bilddaten des Kopfes des Brillenträgers erzeugt und aus diesen die optischen Zentrierparameter ermittelt. Hierbei kann der Blick des Brillenträgers in Gebrauchsstellung z.B. dadurch festgelegt werden, dass der Proband seine Nasenwurzel in einem Spiegelbild fixiert. Ebenso ist es möglich, ein Speckle-Muster bzw. einen leuchtenden Punkt einzusetzen. Dabei ist es ein Ziel, den Blick des Brillenträgers so auszurichten, dass die tatsächliche Ausrichtung der Augen dem zu vermessenden Blickverhalten entspricht.

Aus dem Dokument CN 100 412 646 C ist eine Lichtversorgungsvorrichtung mit einer Leuchtdiode als Punktlichtquelle bekannt. Die Lichtversorgungsvorrichtung umfasst eine reflektierende gekrümmte Oberfläche, eine oder eine Vielzahl von Lichtquelle(n), wobei die Punktlichtquelle an einer seitlichen Stelle der reflektierenden gekrümmten Oberfläche positioniert ist. Die Lichtversorgungsvorrichtung wird verwendet als Lichtquelle der Hintergrundbeleuchtung für verschiedene Flachbildschirme.

Aus der DE 10 2008 003 906 B4 ist ein Fixationstarget als Hilfe zum Ausrichten der Blickrichtung des Brillenträgers für eine solche Zentriervorrichtung bekannt. Das Fixationstarget erzeugt dabei ein Lichtfeld zum Steuern des Blicks des Brillenträgers, während von der Zentriervorrichtung Bilddaten des Kopfes des Brillenträgers erzeugt werden. Dies ist insbesondere bei Fehlsichtigen hilfreich, welche übliche Sehaufgaben z.B. wegen hoher Fehlsichtigkeit und/oder Schielen nicht lösen können.

Als Fixationstarget werden in der technischen Optik justierbare Elemente wie z.B. Linsenhalter eingesetzt, mit denen das Gesamtsystem so eingestellt werden kann, dass das optische System die gewünschten Anforderungen erfüllt. Hierbei kann insbesondere mit hoch-präzisen (und deswegen teuren) Einzelelemente wie z.B. Linsen, Tuben, Blenden usw. gearbeitet werden, um das Lichtfeld des Fixationstargets auszurichten.

Alternativ (oder zusätzlich) zur Verwendung von solchen hoch-präzisen Einzelelementen erfordern die vorbekannten Fixationstargets zumindest ein aufwendiges opto-mechanisches Systems mit Einzelelementen (wie z.B. Mikrometerschrauben), die nur zur Justage des Fixationstargets erforderlich sind. Um das vom Fixationstarget erzeugte Lichtfeld präzise auszurichten, ist hierbei eine aufwändige manuelle Justage des Gesamtsystems erforderlich, welche zeitaufwändig und kostenintensiv ist.

Damit sind die Kosten der vorbekannten Fixationstargets hoch, da dessen Einzelkomponenten hoch präzise ausgebildet sein müssen und/oder aufwändig justiert werden müssen. Insbesondere bei der Linse des Fixationstargets lässt sich die notwendige Präzision hinsichtlich Brennweite, lateraler Lage des Zentrums und/oder Keilfehler nicht mit günstigen Fertigungsverfahren erzielen.

### ZUSAMMENFASSUNG DER ERFINDUNG

Es ist eine Aufgabe der Erfindung, ein kosteneffizientes Fixationstarget für eine Zentriervorrichtung zu ermöglichen.

Diese Aufgabe wird durch die Gegenstände der unabhängigen Ansprüche gelöst. Bevorzugte Ausführungsform sind Gegenstand der abhängigen Ansprüche.

Vor der nachfolgenden, detaillierten Darstellung der Erfindung werden einige Begriffe definiert bzw. beschrieben, welche zum Verständnis der Erfindung beitragen.

Brillengläser sind beispielsweise Einstärkenbrillengläser, Mehrstärkenbrillengläser, beispielsweise Gleitsichtgläser, mit oder ohne Tönung, Verspiegelung und/oder Polarisationsfiltern.

Zwei "Bildaufnahmeeinrichtungen" sind beispielsweise zwei digitale Kameras, welche getrennt voneinander positioniert sind. Es ist möglich, dass eine Bildaufnahmeeinrichtung vorzugsweise eine digitale Kamera und zumindest ein optisches Umlenkelement bzw. -spiegel umfasst, wobei Bilddaten eines Teilbereichs eines Kopfes mit der Kamera mittels des Umlenkspiegels aufgezeichnet bzw. erzeugt werden. Zwei Bildaufnahmeeinrichtungen umfassen daher in gleicher Weise beispielsweise zwei insbesondere digitale Kameras und zumindest zwei Umlenkelemente und/oder -spiegel, wobei jeweils eine digitale Kamera und zumindest ein Umlenkspiegel eine Bildaufnahmeeinrichtung darstellen. Weiterhin vorzugsweise können zwei Bildaufnahmeeinrichtungen auch aus genau einer digitalen Kamera und zwei Umlenkelementen und/oder -spiegeln bestehen, wobei Bilddaten mittels der digitalen Kamera zeitversetzt aufgezeichnet und/oder erzeugt werden. Beispielsweise werden zu einem ersten Zeitpunkt Bilddaten erzeugt, wobei ein Teilbereich eines Kopfes mittels des einen Umlenkspiegels abgebildet wird, und zu einem zweiten Zeitpunkt Bilddaten erzeugt, welche den Teilbereich des Kopfes mittels des anderen Umlenkspiegels abbilden. Ferner kann die Kamera auch derart angeordnet sein, dass an dem ersten und/oder dem zweiten Zeitpunkt von der Kamera Bilddaten erzeugt werden, wobei kein Umlenkspiegel notwendig und/oder zwischen der Kamera und dem Kopf angeordnet ist. Die beiden Bildaufnahmeeinrichtungen können unter verschiedenen Aufnahmerichtungen Bilddaten erzeugen.

Unter zwei unterschiedlichen und/oder verschiedenen "Aufnahmerichtungen" wird verstanden, dass von überlappenden Teilbereichen des Kopfes, vorzugsweise von ein und demselben Teilbereich des Kopfes, verschiedene Bilddaten erzeugt werden, insbesondere, dass Bilddaten und/oder Vergleichsbilddaten von identischen Teilbereichen des Kopfes des Benutzers unter verschiedenen perspektivischen Ansichten erzeugt werden. Folglich wird zwar derselbe Teilbereich des Kopfes abgebildet, die Bilddaten und/oder Vergleichsbilddaten unterscheiden sich jedoch. Unterschiedliche Aufnahmerichtungen können beispielsweise auch dadurch erreicht werden, dass die Bilddaten von zumindest zwei Bildaufnahmeeinrichtungen erzeugt werden, wobei effektive optische Achsen der zumindest zwei Bildaufnahmeeinrichtungen nicht parallel sind.

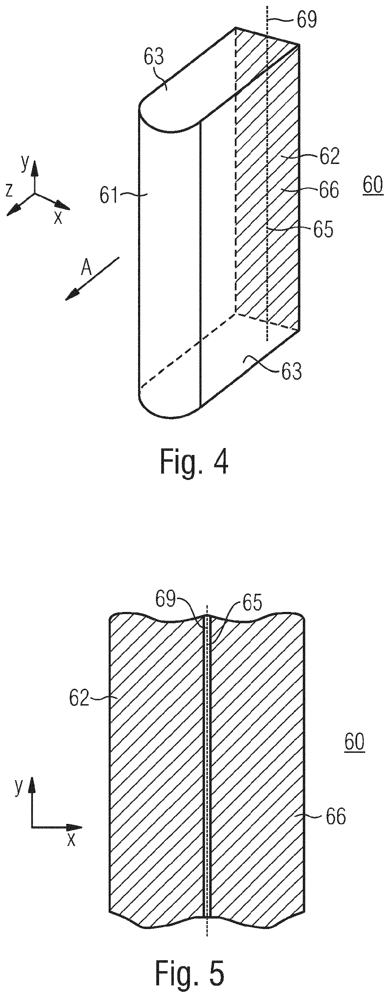

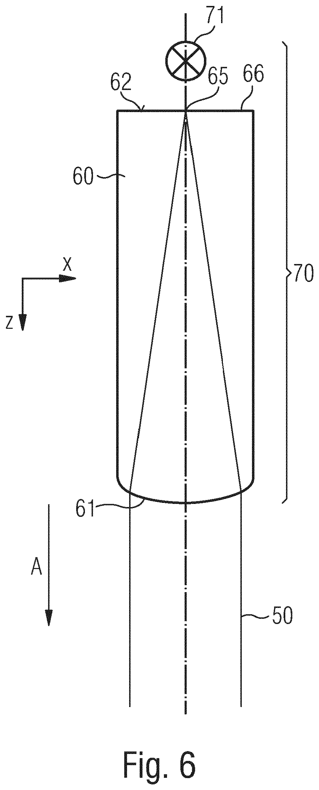

Unter einer Bemaßung im Kastenmaß wird das Maßsystem verstanden, wie es in einschlägigen Normen, beispielsweise in der DIN EN ISO 8624 und/oder der DIN EN ISO 13666 und/oder der DIN 58 208 und/oder der DIN 5340, beschrieben wird. Ferner wird hinsichtlich des Kastenmaßes und weiterer verwendeter herkömmlicher Begriffe und Parameter auf das Buch "Die Optik des Auges und der Sehhilfen" von Dr. Roland Enders, 1995 Optische Fachveröffentlichung GmbH, Heidelberg, sowie das Buch "Optik und Technik der Brille" von Heinz Diepes und Ralf Blendowske, 2002 Verlag Optische Fachveröffentlichungen GmbH, Heidelberg, verwiesen. Ebenso wird auch auf die Broschüre "inform fachberatung für die augenoptik" PR-Schriftenreihe des ZVA für den Augenoptiker, Heft 9, "Brillenzentrierung", ISBN 3-922269-23-0, 1998 verwiesen, in welcher das Kastenmaß insbesondere in Fig. 5 und Fig. 6 beispielhaft dargestellt ist. Weiterhin wird auch auf das Buch "Brillenanpassung Ein Schulbuch und Leitfaden" von Wolfgang Schulz und Johannes Eber 1997, DOZ-Verlag, herausgegeben vom Zentralverband der Augenoptiker, Düsseldorf, ISBN 3-922269-21-4 verwiesen, insbesondere auf Punkte 1.3, 1.4. und 1.5 und die zugehörigen Abbildungen. Die Normen, die genannte Broschüre sowie die genannten Bücher stellen für die Begriffsdefinitionen insoweit einen integralen Offenbarungsbestandteil der vorliegenden Anmeldung dar.

Die "Pupillendistanz" entspricht im Wesentlichen dem Abstand der Pupillenmitten, insbesondere in Nullblickrichtung.

Der Augendrehpunkt eines Auges ist der Punkt des Auges, der bei einer Bewegung des Auges, bei festgelegter Kopfhaltung, beispielsweise einer Blicksenkung oder Blickhebung durch Rotation des Auges im Wesentlichen in Ruhe bleibt. Der Augendrehpunkt ist somit im Wesentlichen das Rotationszentrum des Auges.

Effektive optische Achsen der Bildaufnahmeeinrichtungen sind diejenigen Bereiche von Linien, welche von dem Mittelpunkt der jeweiligen Aperturen der Bildaufnahmeeinrichtungen senkrecht zu diesen Aperturen ausgehen und den abgebildeten Teilbereich des Kopfes des Benutzers schneiden. In anderen Worten handelt es sich bei den effektiven optischen Achsen insbesondere um die optischen Achsen der Bildaufnahmeeinrichtungen, wobei diese optischen Achsen herkömmlicherweise senkrecht zu einem Linsensystem der Bildaufnahmeeinrichtungen angeordnet sind und vom Zentrum des Linsensystems ausgehen. Befinden sich im Strahlengang der Bildaufnahmeeinrichtungen keine weiteren optischen Elemente, wie beispielsweise Umlenkspiegel oder Prismen, so entspricht die effektive optische Achse im Wesentlichen der optischen Achse der Bildaufnahmeeinrichtung. Sind jedoch im Strahlengang der Bildaufnahmeeinrichtung weitere optische Elemente, beispielsweise ein oder mehrere Umlenkspiegel, angeordnet, entspricht die effektive optische Achse nicht mehr der optischen Achse der Bildaufnahmeeinrichtung, wie sie von der Bildaufnahmeeinrichtung ausgeht.

Anders ausgedrückt ist die effektive optische Achse derjenige Bereich einer gegebenenfalls mehrfach optisch umgelenkten optischen Achse einer Bildaufnahmeeinrichtung, welcher ohne Änderung der Richtung den Kopf des Benutzers schneidet. Die optische Achse der Bildaufnahmeeinrichtung entspricht einer Linie, welche von einem Mittelpunkt einer Apertur der Bildaufnahmeeinrichtung unter einem rechten Winkel zu einer Ebene, welche die Apertur der Bildaufnahmeeinrichtung umfasst, ausgeht, wobei die Richtung der optischen Achse der Bildaufnahmeeinrichtung durch optische Elemente, wie beispielsweise Spiegel und/oder Prismen, veränderbar ist. Die effektiven optischen Achsen zweier Bildaufnahmeeinrichtungen können sich beinahe schneiden.

Eine "Zylinderlinse" ist eine Linse, deren gekrümmten Flächen zumindest teilweise als zumindest ein Ausschnitt einer Zylinderfläche ausgebildet sind oder solchen Ausschnitten von Zylinderflächen ähneln. Im Gegensatz zu einer sphärischen Linse, die Licht auf einen einzigen Punkt fokussiert, fokussiert die Zylinderlinse einen Lichtstrahl längs einer einzigen Achse, der "Brennachse" und/oder "Brennlinie". Mathematisch kann eine zylindrische Linse entsprechend einer sphärischen Linse beschrieben werden, jedoch nur in einer Ebene. Eine Zylinderlinse kann auch als Azylinder oder auch asphärischer Zylinder ausgebildet sein, d.h. als Linse mit zylindrischer Oberfläche, deren Querschnitt von der Kreisform abweicht. Plankonkave und plankonvexe Azylinder können genauso verwendet werden wie solche mit sphärischer oder asphärischer Rückseite. Eine solche asphärische Zylinderlinse kann einfallendes Licht entlang einer Fokuslinie ohne die Einflüsse der sphärischen Aberration bündeln.

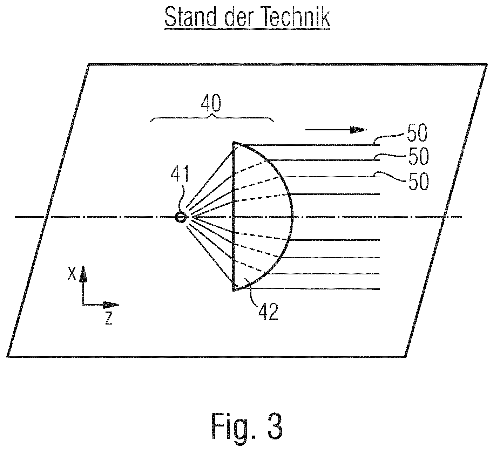

Die "optische Achse" eines Fixationstargets mit einer Zylinderlinse ist eine Achse, die parallel zu einer Richtung in der Brennlinie erzeugter elektromagnetischer Strahlen ist, die nach Durchgang durch die Zylinderlinse parallel sind (vgl. hierzu auch die Ausbreitungsrichtung der in Fig. 3 gezeigten, parallelen Lichtstrahlen 50).

Der Begriff "im Wesentlichen parallel" beschreibt elektromagnetische Strahlung, deren Ausbreitungsrichtung insbesondere parallel ist. Das heißt zwei elektromagnetische Strahlen sind parallel, wenn ihre Ausbreitungsrichtungen identisch sind. Dies ist insbesondere der Fall für elektromagnetische Strahlung nach Durchgang durch eine Zylinderlinse, wenn eine Quelle der elektromagnetischen Strahlung in der Brennebene im Wesentlichen parallel zu der Brennlinie der Zylinderlinse, insbesondere in der Brennlinie einer Zylinderlinse angeordnet ist. Sind Quellen elektromagnetischer Strahlung in der Brennlinie angeordnet, ist die Strahlung zugleich senkrecht zur Linsenebene.

Zwei elektromagnetische Strahlen können auch dann im Wesentlichen parallel sein, wenn ihre Ausbreitungsrichtungen einen Winkel miteinander einschließen, wobei dieser Winkel kleiner als etwa 10°, weiterhin vorzugsweise kleiner als etwa 5°, besonders bevorzugt kleiner als etwa 2°, besonders bevorzugt kleiner als etwa 1°, besonders bevorzugt kleiner als etwa 0,25°, besonders bevorzugt kleiner als etwa 0,1°, ganz besonders bevorzugt kleiner als etwa 0,05° ist. Passieren zwei elektromagnetische Strahlen die Brennlinie einer Zylinderlinse und sind die beiden elektromagnetischen Strahlen senkrecht zu der Brennlinie, sind sie nach Durchgang durch die Zylinderlinse im Wesentlichen parallel. Passiert nur einer der Strahlen die Brennlinie und der andere Strahl passiert die Brennlinie nicht oder passieren beide Strahlen die Brennlinie nicht und sind die beiden Strahlen senkrecht zu der Brennlinie, sind die beiden Strahlen nach Durchgang durch die Zylinderlinse im Wesentlichen parallel, wenn der jeweilige Abstand von der Brennlinie kleiner als ein vorgegebener Wert ist. Dies kann beispielsweise dadurch erreicht werden, dass eine Lichtquelle nicht in der Brennlinie angeordnet ist, sondern die Lichtquelle von der Brennlinie beabstandet ist. Vorzugsweise ist der Abstand der Lichtquelle von der Brennlinie (bzw. der Brennebene) kleiner als etwa 5%, vorzugsweise kleiner als etwa 2%, vorzugsweise kleiner als etwa 1%, vorzugsweise kleiner als etwa 0,5%, vorzugsweise kleiner als etwa 0,1% der Brennweite der Zylinderlinse. Vorteilhafterweise ermöglicht die Vorrichtung somit für die Bestimmung der Pupillendistanzen vorzugsweise eine Messgenauigkeit von zumindest etwa ±0,2 mm, bevorzugt von zumindest etwa ±0,05 mm, weiterhin bevorzugt von zumindest etwa ±0,01 mm. Dies entspricht für ein Gullstrand-Auge (Radius 12 mm) einer Winkelauslenkung des Auges von weniger als ca. ±1°. Diese Auslenkung wir durch eine gleich große Abweichung zwischen der Soll-Richtung der optischen Achse des Targets und deren tatsächlicher Richtung hervorgerufen. Somit wird für den oben genannten Abstand der Lichtquelle von der Brennlinie vorzugsweise eine Abweichung der Winkelauslenkung des Auges kleiner als etwa 1° ermöglicht.

Die Begriffe "elektromagnetische Strahlung", "Licht" und "Lichtstrahlen" können synonym verwendet werden.

Der Begriff "im Wesentlichen" kann eine geringfügige Abweichung von einem Sollwert beschreiben, insbesondere eine Abweichung im Rahmen der Herstellungsgenauigkeit und/oder im Rahmen der notwendigen Genauigkeit, so dass ein Effekt beibehalten wird, wie er bei dem Sollwert vorhanden ist. Der Begriff "im Wesentlichen" kann daher eine Abweichung von weniger als etwa 30%, weniger als etwa 20%, weniger als etwa 10%, weniger als etwa 5%, weniger als etwa 2%, bevorzugt weniger als etwa 1% von einem Sollwert und/oder Sollposition, usw. beinhalten. Der Begriff "im Wesentlichen" umfasst den Begriff "identisch", d. h. ohne Abweichung von einem Sollwert, einer Sollposition usw. sein.

Der Begriff "Lichtfeld" beschreibt elektromagnetische Strahlung, die von einem flächigen Objekt ausgestrahlt wird. Das flächige Objekt kann beispielsweise Bestandteil eines Fixationstargets sein. Das flächige Objekt kann beispielsweise eine gekrümmte Fläche einer Zylinderlinse sein, durch die elektromagnetische Strahlung aus der Zylinderlinse austritt. Obwohl in diesem Fall die elektromagnetische Strahlung durch die gekrümmte Oberfläche austritt, empfindet ein Brillenträger, der das Lichtfeld betrachtet, das Lichtfeld beispielsweise als von einem ebenen, d. h. nicht gekrümmten flächigen Objekt ausgestrahlt. Das Lichtfeld kann auch von einer Fläche eines Diffusors ausgestrahlt werden, die beispielsweise rechteckig ist. In anderen Worten beschreibt ein "im Wesentlichen rechteckiges Lichtfeld" in seiner allgemeinsten Form ein Lichtfeld mit einer Längsausdehnung und einer Breitenausdehnung, wobei die Längsausdehnung z.B. größer sein kann als die Breitenausdehnung. Es ist auch möglich, dass das Lichtfeld im Wesentlichen quadratisch ist, d. h. die Längsausdehnung in etwa gleich der Breitenausdehnung ist. Folglich kann das im Wesentlichen rechteckige Lichtfeld die elektromagnetische Strahlung sein, die von einer im Wesentlichen rechteckigen Fläche ausgestrahlt wird, beispielsweise einer zumindest teilweise lichtdurchlässigen von hinten beleuchteten Fläche. Insbesondere kann ein im Wesentlichen rechteckiges Lichtfeld ein Lichtfeld sein, dessen Projektion auf eine Projektionsebene im Wesentlichen ein Rechteck ist, wobei die Projektionsebene senkrecht zu den elektromagnetischen Strahlen ist die parallel zueinander sind, d. h. die Projektionsebene ist im Wesentlichen senkrecht zu der zweiten Ebene (s. u.). Der Begriff "im Wesentlichen rechteckig" beinhaltet auch Abweichungen von der Rechteckform, z. B. mit abgerundeten Ecken, im Wesentlichen ellipsenförmig, insbesondere mit einem Verhältnis der langen Halbachse zu der kurzen Halbachse von mehr als 1:2. Um zu vermeiden, dass der Brillenträger bei einem elliptischen Target von der habituellen Kopf- und Körperhaltung abweicht, um ein möglichst langes Target zu betrachten, ist das Target vorzugsweise rechteckig.

Eine "Linie" ist nicht auf eine Linie im mathematischen Sinn beschränkt. Vielmehr umfasst der Begriff Linie auch ein zweidimensionales Objekt mit einer endlichen Länge und einer endlichen Breite. Eine Linie kann somit ein Rechteck mit einer geringen Breite im Vergleich zu der Länge des Rechtecks sein.

Der Begriff "homogenes Licht" insbesondere entlang einer Richtung beschreibt, dass insbesondere entlang dieser Richtung von der Beleuchtungseinrichtung Licht mit im Wesentlichen gleicher Lichtleistung und/oder Leuchtkraft ausgestrahlt wird. An allen Punkten der Beleuchtungseinrichtung entlang dieser Richtung, von denen Licht ausgestrahlt wird, weist das ausgestrahlte Licht eine zumindest ähnliche, strukturfreie Intensität auf. Die Intensität kann dabei zu den Rändern hin z.B. etwas geringer werden. Wenn das ausgestrahlte Licht in dieser Richtung im Wesentlichen homogen ist, kann der Betrachter keine einzelnen Lichtquellen differenzieren, sondern nimmt eine leuchtende Linie und/oder aufgrund der endlichen Ausdehnung der Beleuchtungseinrichtung, einen leuchtenden Streifen und/oder eine leuchtende Fläche war, der und/oder die Licht einheitlicher Intensität ausstrahlt. Dies gilt für eine Vielzahl von Richtungen, insbesondere für eine Lichtabstrahlfläche.

Der Begriff "habituelle Kopf- und Körperhaltung" stellt die Basis einer exakten und verträglichen Brillenglaszentrierung dar. Insbesondere entspricht die "habituelle Kopf- und Körperhaltung" im Wesentlichen einer möglichst natürlichen Kopf- und Körperhaltung des Brillenträgers. Der Brillenträger kann die "habituelle Kopf- und Körperhaltung" beispielsweise einnehmen, wenn er sich selbst im Spiegel betrachtet, da das Betrachten im Spiegel für jeden Menschen eine alltägliche und sehr gewohnte Situation darstellt. Beispielsweise kann eine habituelle Kopf- und Körperhaltung, verglichen mit einem natürlichen Blick in die Ferne, erreicht werden, wenn der Proband seine Nasenwurzel in dem Spiegelbild fixiert.

Insbesondere kann die habituelle Kopf- und Körperhaltung der natürlichen Haltung des Brillenträgers entsprechen, welche durch seine körperliche und psychische Befindlichkeit, Gewohnheit, Alltag, Beruf und Freizeit bestimmt wird.

Eine entspannte Nackenhaltung und eine gesunde, im Wesentlichen ideale Kopfhaltung hat der Brillenträger insbesondere dann, wenn der Kopf genau über den Schultern (und in der Verlängerung nach unten genau über dem Fußgewölbe) positioniert ist. Somit wird die habituelle Kopf- und Körperhaltung vorzugsweise im Stehen eingenommen.

Bei im Wesentlichen idealer Kopfhaltung sitzt der Kopf im Wesentlichen genau über den Schultern (und in der Verlängerung nach unten genau über dem Fußgewölbe). Die Ohren stehen senkrecht und befinden sich über der Mitte der Schultern. Der Nacken ist nur ganz leicht konkav, also einwärts gewölbt. In dieser Position wird das Gewicht des Kopfes über die Wirbelsäule vom ganzen Skelett, also von den Knochen getragen. Da die Nackenmuskeln keinerlei Gewicht tragen brauchen, sind sie allesamt weich und der Kopf ist auf der Wirbelsäule frei beweglich. Bei allen anderen Kopf- und/oder Nackenhaltungen sind die Nackenmuskeln chronisch angespannt, denn sie müssen nun das Gewicht des Kopfes gegen die Schwerkraft halten. Je nachdem, ob der Kopf nach vorn oder hinten gezogen ist oder nach rechts oder links geneigt gehalten wird, und ob der Nacken dabei stärker oder weniger gekrümmt ist, befinden sich unterschiedliche Nacken- und Körpermuskeln in Dauerkontraktion, sind also unterschiedliche Muskeln verspannt. Das führt zu unterschiedlichen Kopf- und Nackenschmerzen. Gleichzeitig ist die Beweglichkeit des Nackens eingeschränkt, da die Muskeln den Kopf in einer bestimmten Haltung fixieren müssen und daher nur in eingeschränktem Umfang für Bewegung zur Verfügung stehen.

Ein Aspekt betrifft ein Fixationstarget zum Erzeugen eines Lichtfelds zum Ausrichten der Blickrichtung eines Brillenträgers bei Vermessung des Brillenträgers mittels einer Zentriervorrichtung. Das Fixationstarget weist einen transparenten Linsenkörper auf, mittels welchem im Betrieb eine elektromagnetische Strahlung des erzeugten Lichtfelds optisch geformt wird. Aus einer optischen Abstrahlseite des Linsenkörpers tritt im Betrieb die elektromagnetische Strahlung des erzeugten Lichtfelds in eine Abstrahlrichtung aus. Eine Rückseite des Linsenkörpers ist an einer der Abstrahlseite abgewandten Rückseitenfläche des Linsenkörpers ausgebildet. Dabei erstreckt sich der transparente Linsenkörper von der Abstrahlseite entgegen der Abstrahlrichtung bis zur Rückseite und dabei bis zumindest zu einer Brennebene des Linsenkörpers.

Das Fixationstarget ist dazu ausgebildet, im Betrieb ein Lichtfeld abzustrahlen, welches in einer horizontalen Ebene parallel zur Ausbreitungsrichtung und diffus in einer vertikalen Richtung ausgebildet ist. Dadurch kann ein Auge im Bereich des Lichtfelds in horizontaler Richtung parallel zur Ausbreitungsrichtung des Lichtfeld ausgelenkt werden, ohne in vertikaler Richtung beeinflusst zu werden. Wird ein solches Fixationstarget in einer Zentriervorrichtung eingesetzt, kann die Ausbreitungsrichtung so ausgerichtet werden, dass das Lichtfeld von der Zentriervorrichtung zum Brillenträger strahlt. Fixiert der Brillenträger bei einer Vermessung das Lichtfeld des Fixiertargets, so kann seine Augenstellung vom Lichtfeld eingestellt und/oder gesteuert werden.

Um während der Vermessung eine Fehlauslenkung des oder der Augen zu vermeiden, kann die horizontale Komponente der Richtung des Lichtfelds über den ganzen Bereich gleichmäßig parallel ausgebildet sein. Andernfalls würde das Auge in der horizontalen Richtung abweichend von Richtung des Lichtfelds ausgelenkt, nämlich in der jeweiligen lokalen Richtung des Lichtfeldes am Ort der Pupille.

Das Fixationstarget kann mit seinem Lichtfeld zumindest einen Messort beleuchten, an welchem zumindest ein Auge des Brillenträgers angeordnet werden kann.

Das Fixationstarget weist eine optische Linse auf, welche von dem transparenten Linsenkörper gebildet wird. Der transparente Linsenkörper kann zum Beispiel aus einem Glas und/oder einem Kunststoff ausgebildet sein. Der transparente Linsenkörper formt die elektromagnetische Strahlung durch seine Form optisch und wirkt dadurch als eine Linse. Der transparente Linsenkörper kann insbesondere die Form und/oder die optische Wirkung einer (z.B. sphärische oder asphärischen) Zylinderlinse mit einer in der Brennebene angeordneten Brennlinie aufweisen. Alternativ kann der transparente Linsenkörper die Form und/oder die optische Wirkung einer sphärischen Linse mit einem in der Brennebene angeordneten Brennpunkt aufweisen.

Das Fixationstarget kann neben dem transparenten Linsenkörper auch eine Lichtquelle aufweisen, deren elektromagnetische Strahlung von dem transparenten Linsenkörper optisch so geformt wird, dass sie nach Austritt aus der Abstrahlseite das Lichtfeld des Fixationstarget ausbildet.

Die optische Abstrahlseite des Linsenkörpers kann zum Beispiel zumindest teilweise konvex ausgebildet sein und/oder zumindest teilweise konkav. Die Abstrahlseite kann sphärisch oder asphärisch ausgebildet sein. Die Abstrahlseite kann in einer Betriebsposition dem Brillenträger zugewandt angeordnet werden. So kann das aus der Abstrahlseite austretende Lichtfeld zur Ausrichtung des Blicks des Brillenträgers dienen.

Die optische Abstrahlseite des Linsenkörpers kann die oder zumindest eine Seite des transparenten Linsenkörpers ausbilden, welche durch ihre Form elektromagnetische Strahlung bündelt und/oder optisch so formt, dass das vorbestimmte und/oder gewünschte Lichtfeld erzeugt wird.

Die Rückseite des Linsenkörpers, welche an dem der Abstrahlseite abgewandten Ende des transparenten Linsenkörpers ausgebildet ist, kann im Wesentlichen flach und/oder plan ausgebildet sein.

Die Position der Brennebene ist eine andere für einen transparenten Linsenkörper, der die Brennebene umfasst, als für einen, der vor der Brennebene endet. Dies liegt darin begründet, dass in ersterem Fall nur eine Brechung an der Austrittsfläche auftritt, während bei kleineren transparenten Linsenkörper zwei Brechungen auftreten, nämlich bei Eintritt in die Rückseite und bei Austritt an der Austrittsfläche.

Im Gegensatz zu vorbekannten Fixationstargets kann der transparente Linsenkörper zum Beispiel monolithisch ausgebildet sein. Der transparente und massive Linsenkörper ist entgegen der Abstrahlungsrichtung verlängert ausgebildet. Deswegen erstreckt er sich von der Abstrahlseite entgegen der Abstrahlrichtung zumindest bis zur Brennebene des Linsenkörpers. Die Brennebene des Linsenkörpers kann zumindest abschnittsweise oder vollständig an der Rückseite des Linsenkörpers ausgebildet sein. Die Brennebene des Linsenkörpers kann zumindest abschnittsweise oder sogar vollständig im Inneren des Linsenkörpers ausgebildet sein. Theoretisch kann die Brennebene auch abschnittsweise im Inneren des Linsenkörpers und abschnittsweise an der Rückseite ausgebildet sein.

Die Brennebene ist dabei diejenige Ebene, auf welche der transparente Linsenkörper Lichtstrahlen fokussiert. Je nach Linsentyp kann der Linsenkörper Lichtstrahlen z.B. in einer Brennlinie oder in einem Brennpunkt fokussieren. Eine in der Brennlinie angeordnete leuchtende Linie erzeugt elektromagnetische Strahlung, welche nach Austritt aus der Abstrahlseite zumindest in einer Richtung im Wesentlichen parallel zueinander ausgebildet ist. Ähnlich dazu erzeugt eine im Brennpunkt angeordnete punktförmige Lichtquelle elektromagnetische Strahlung, welche nach Austritt aus der Abstrahlseite im Wesentlichen parallel zueinander ausgebildet ist.

Die entgegen der Abstrahlrichtung verlängerte Form des transparenten Linsenkörpers ermöglicht es, einen für eine Lichtquelle des Fixationstargets vordefinierten Anordnungsplatz bereitzustellen. Dies vereinfacht die Anordnung der Lichtquelle relativ zum Linsenkörper. Die Justage und/oder das Aufbauen des Fixationstarget wird dadurch vereinfacht. Die längliche Form des Linsenkörpers kann somit einen Fehlaufbau verhindern und/oder zumindest die Gefahr eines Fehlaufbaus reduzieren.

Ein weiterer Vorteil kann eine erhöhte Stabilität des Fixationstargets sein, da es weniger Einzelteile aufweist und diese dadurch weniger anfällig für eine Dejustage sein können.

Entlang zumindest eines Abschnitts der Brennebene ist am und/oder im Linsenkörper ein Lichtgenerator ausgebildet. Vom Lichtgenerator wird im Betrieb eine elektromagnetische Strahlung in Richtung zur optischen Abstrahlseite hin derart abgestrahlt, dass diese elektromagnetische Strahlung nach dem Austritt aus der Abstrahlseite das vom Fixationstarget erzeugte Lichtfeld ausbildet. Der Lichtgenerator ist somit vorinstalliert am und/oder im Linsenkörper. Damit kann eine Fehljustage der Lichtquelle relativ zum Linsenkörper deutlich reduziert oder sogar vollständig vermieden werden. Der Lichtgenerator kann z.B. als ein Lichtliniengenerator ausgebildet sein und eine etwa linienförmige Lichtquelle bereitstellen. Nicht wie in der beanspruchten Erfindung kann der Lichtgenerator z.B. als eine etwa punktförmige Lichtquelle ausgebildet sein, also als ein Lichtpunktgenerator. Mit "Lichtpunktgenerator" ist keine mathematisch (also infinitesimal kleine) Punktlichtquelle gemeint, sondern eine technisch realisierbare, etwa kugelförmige kleine Lichtquelle. Der Lichtgenerator muss nicht selbst als eine aktive, also energiebetriebene, Lichtquelle ausgebildet sein. Der Lichtgenerator kann die Lichtquelle zum Beispiel mittels Beugung und/oder Streuung simulieren. Dabei kann der Lichtgenerator die elektromagnetische Strahlung, welche tatsächlich von einer anderen z.B. externen elektrischen Lichtquelle erzeugt wird, so optisch manipulieren, dass er wie eine eigene Lichtquelle wirkt.

In einer Ausführungsform ist der Lichtgenerator als ein passiver Lichtgenerator ausgebildet, welche nur unter Beleuchtung durch eine Lichtquelle die elektromagnetische Strahlung des Lichtfelds bereitstellt und/oder generiert. Als passives Bauteil ist der Lichtgenerator nicht selbst energiebetrieben. Er kann selbst keine Lichtstrahlen erzeugen, sondern nur bereits vorhandene Lichtstrahlen beeinflussen. Hierbei kann der Lichtgenerator zum Beispiel als ein Beugungsspalt, eine Lochblende und/oder als eine Linie von Streupunkten ausgebildet sein. Wird der Lichtliniengenerator durch eine aktive Lichtquelle wie zum Beispiel zumindest eine Glühbirne und/oder LED beleuchtet, so bildet er eine leuchtende Linie aus, welche genau in der Brennlinie und/oder Brennebene des Linsenkörpers diffus Licht abstrahlt. Der passive Lichtgenerator kann auch durch zumindest einen phosphoreszierenden Farbstoff ausgebildet sein, welcher unter

Beleuchtung mittels z.B. einer UV-Lichtquelle die elektromagnetische Strahlung des Lichtfelds generiert.

Das Fixationstarget weist eine Lichtquelle zur Beleuchtung des Lichtgenerators derart auf, dass der Lichtgenerator eine von der Lichtquelle emittierte elektromagnetische Strahlung formt und als elektromagnetische Strahlung in Richtung zur optischen Abstrahlseite hin derart abstrahlt, dass diese elektromagnetische Strahlung nach dem Austritt aus der Abstrahlseite das vom Fixationstarget erzeugte Lichtfeld ausbildet. Die Lichtquelle kann eine aktive, energiebetriebene Lichtquelle sein. Sie kann z.B. als zumindest eine LED und/oder Glühbirne und/oder Halogenleuchte oder als ein ähnliches, strombetriebenes Leuchtmittel ausgebildet sein. Der Lichtgenerator ist somit als ein aktiver Lichtgenerator ausgebildet im Gegensatz zum passiven Lichtgenerator. Da der in der Brennebene angeordnete Lichtgenerator z.B. als Lichtliniengenerator oder Lichtpunktgenerator das von der Lichtquelle erzeugte Licht optisch umformt zu z.B. einer leuchtenden Linie oder einem Lichtpunkt, muss das Licht der Lichtquelle relativ zum Linsenkörper nicht besonders genau justiert und/oder ausgerichtet sein. Der Lichtgenerator vereinfacht somit das Assemblieren des Fixationstargets erheblich.

Gemäß einer Ausführungsform ist die Brennebene zumindest abschnittsweise genau an der Rückseite des Linsenkörpers angeordnet. Hierbei kann die Rückseite flach und/oder plan ausgebildet sein. Die Rückseite kann in Betriebsposition insbesondere in einer vertikalen Ebene angeordnet sein. Weist der Linsenkörper eine Brennlinie auf, so kann auch die Brennlinie in Betriebsposition etwa vertikal ausgerichtet sein. Soll nun eine Lichtquelle des Fixationstargets, wie zum Beispiel eine leuchtende Linie, assembliert werden, so kann sie einfach an die Rückseite angelegt werden, und dort kann sie in die Brennlinie verschoben werden. Entlang der Brennlinie kann auf der Rückseite eine Brennlinienmarkierung ausgebildet sein. Gleiches kann auch für eine punktförmige Lichtquelle mittels einer Brennpunktmarkierung vorgesehen sein.

Gemäß einer Weiterbildung ist der Lichtgenerator dadurch bereitgestellt, dass die Rückseite des Linsenkörpers bis auf zumindest einen Abschnitt und/oder Bereich entlang der Brennebene geschwärzt und/oder aufgeraut ausgebildet ist. Hierbei kann die Rückseite zum Beispiel vollständig bis auf den z.B. spaltförmigen Abschnitt entlang der Brennlinie oder z.B. einen kreisförmigen Bereich um den Brennpunkt geschwärzt und/oder aufgeraut ausgebildet sein. Wird die Rückseite beispielsweise mittels einer LED als aktive Lichtquelle beleuchtet, so entsteht durch den Linsenkörper das Lichtfeld, welches als Fixationstarget genutzt werden kann. Die Aufrauhung und/oder Schwärzung der Rückfläche verhindert dabei Mehrfachreflexionen. Die Schwärzung erzeugt eine z.B. spaltförmige oder kreisförmige Blende für das Beleuchtungslicht. Der Spalt entlang der Brennlinie kann z.B. schmäler als etwa 1 cm ausgebildet sein, bevorzugt schmäler als etwa 5 mm, besonders bevorzugt schmäler als etwa 1 mm. Als besonders geeignet hat sich eine Spaltbreite von etwa 0,5 mm erwiesen. Dieselben Maße gelten bevorzugt für mögliche Durchmesser einer kreisförmigen Blende.

In einer alternativen Ausführungsform ist die Brennebene zumindest abschnittsweise im Inneren des Linsenkörpers angeordnet. Die Brennebene wird hierbei von dem Linsenkörper eingeschlossen. Deswegen ist alles, was an der Brennebene angeordnet ist, wie z.B. ein Lichtgenerator, bereits ein Bestandteil des Linsenkörpers und muss nicht mehr justiert werden. Hierbei ist der Linsenkörper entgegen der Abstrahlrichtung über die Brennebene hinaus ausgebildet. Alternativ kann entlang und/oder in der Brennebene im Inneren des Linsenkörpers eine passgenaue Aussparung für z.B. eine aktiv leuchtende Linie ausgebildet sein, welche beim Assemblieren lediglich in diese Aussparung eingeschoben wird.

Bei einer Weiterbildung dieser Ausführungsform ist der Lichtgenerator dadurch bereitgestellt, dass im Inneren des Linsenkörpers entlang zumindest eines Abschnitts der Brennebene Streuzentren ausgebildet sind. Die Streuzentren können zum Beispiel mittels eines Laserschreibverfahrens in das Material des Linsenkörpers eingebracht werden. Die Streuzentren können ähnlich zu einer Glasinnengravur erzeugt werden. Um sicherzustellen, dass die Streuzentren im Volumen des Linsenkörpers exakt entlang der Brennlinie oder im Brennpunkt angeordnet sind, kann die Erzeugung mithilfe eines Lichtbündels mit der für das Lichtfeld gewünschten Eigenschaft erfolgen. Hierbei können die Lichtbündel typischerweise in sich parallel und parallel zur optischen Achse des Fixationstargets in die Abstrahlseite hinein eingestrahlt werden. Dadurch werden sie automatisch auf der Brennlinie oder im Brennpunkt fokussiert und erzeugen dort die Streuzentren.

Gemäß einer Ausführungsform ist zumindest eine Außenfläche des Linsenkörpers zumidnest teilweise geschwärzt und/oder aufgeraut ausgebildet. Bei diesen Außenflächen kann es sich insbesondere um laterale Außenflächen des Linsenkörpers handeln, welche sich entlang einer Seitenfläche erstrecken, zum Beispiel von der Abstrahlseite zur Rückseite und/oder von einer Oberseite bis zu einer Unterseite. Durch die Schwärzung und/oder Aufrauhung können z.B. Reflexionen innerhalb des Linsenkörpers reduziert und/oder vermieden werden. Auch an der Abstrahlseite können z.B. Kanten geschwärzt sein, um ungünstige Reflexionen zu reduzieren.

Gemäß einer Ausführungsform ist zumindest eine Außenfläche des Linsenkörpers mit einer Antireflexbeschichtung beschichtet. Solche Antireflexbeschichtungen werden üblicherweise auf Brillengläser aufgebracht und sind dem Fachmann daher bekannt. Die Antireflexbeschichtungen können auf sämtliche Außenflächen des Linsenkörpers aufgebracht werden, um Reflexionen zu reduzieren und/oder zu vermeiden.

Gemäß einer Ausführungsform ist der transparente Linsenkörper entweder ausgebildet als eine sphärische oder asphärische Zylinderlinse mit einer Brennlinie, welche in der Brennebene angeordnet ist, oder als eine sphärische oder asphärische Linse mit einem Brennpunkt, welcher in der Brennebene angeordnet ist. Hierbei kann die Brennlinie der Zylinderlinse in Betriebsposition etwa vertikal ausgerichtet sein. Die asphärische Form ermöglicht hierbei kompaktere Geometrie des Linsenkörpers und/oder geringere Fehler am Rande des Linsenkörpers. Diese asphärische Form kann jedoch aufwendiger zu fertigen sein als die sphärische Form. Zur Berechnung der Form wird auf dem Fachmann bekannte Methoden verwiesen und z.B. auf die Druckschrift DE 10 2008 003 906 B4 und G. Esser et al.: "Derivation of the refraction equations for higher order aberrations of local wavefronts at oblique incidence", JOSA A, Vol. 27, No. 2 (2010). Ist der Linsenkörper als eine Zylinderlinse ausgebildet, so weist er eine Brennlinie auf und wird bevorzugt mit einem in der Brennlinie angeordneten Lichtliniengenerator kombiniert. Ist der Linsenkörper als sphärische Linse ausgebildet, so weist er einen Brennpunkt auf und wird nicht wie in der beanspruchten Erfindung mit einem im Brennpunkt angeordneten Lichtpunktgenerator kombiniert.

In einer Weiterbildung dieser Ausführungsform ist entweder der Lichtgenerator als ein Lichtliniengenerator ausgebildet, welcher etwa entlang zumindest eines Abschnitts der Brennlinie der Zylinderlinse angeordnet ist, oder es ist der Lichtgenerator als eine punktförmige Lichtquelle ausgebildet, welche etwa im Brennpunkt der Linse angeordnet ist. Je nach Ausgestaltung des Linsenköpers kann der Lichtgenerator z.B. als ein Lichtliniengenerator ausgebildet sein und eine etwa linienförmige Lichtquelle bereitstellen. Nicht wie in der beanspruchten Erfindung kann der Lichtgenerator z.B. als eine etwa punktförmige Lichtquelle ausgebildet sein, also als ein Lichtpunktgenerator. Mit "Lichtpunktgenerator" ist auch hier wiederum keine mathematisch (also infinitesimal kleine) Punktlichtquelle gemeint, sondern eine technisch realisierbare, etwa kugelförmige kleine Lichtquelle. Jedenfalls muss der Lichtgenerator muss nicht selbst als eine aktive, also energiebetriebene, Lichtquelle ausgebildet sein, sondern der Lichtgenerator kann die Lichtquelle zum Beispiel mittels Beugung und/oder Streuung simulieren. Die Anordnung des Lichtgenerators in der Brennlinie bzw. im Brennpunkt ermöglicht der Ausbildung paralleler Lichtstrahlen nach Durchgang durch den Linsenkörper.

Gemäß einer Ausführungsform ist der transparente Linsenkörper aus Kunststoff gegossen und/oder gezogen. Bei dieser Herstellungsmethode kann der transparente Linsenkörper besonders passgenau hergestellt werden. Der Aufbau des Linsenkörpers als Kunststoffprofil erlaubt ausgedehnte Geometrien in vertikaler Ausdehnung, und damit große Bauhöhen. Eine andere Möglichkeit stellt ein klassisches Schleifen bei Kunststoff oder Mineralglas dar.

Gemäß einer Ausführungsform ist das Fixationstarget derart ausgebildet, dass die elektromagnetische Strahlung des Lichtfelds in einer ersten vorbestimmbaren Ebene im Wesentlichen diffus ausgebildet ist, und dass die elektromagnetische Strahlung des Lichtfelds in einer zweiten vorbestimmbaren Ebene, die etwa senkrecht zu der ersten Ebene angeordnet ist, im Wesentlichen parallel ausgebildet ist. In anderen Worten kann der Strahlengang in einer Richtung parallel verlaufen und in der dazu senkrechten Richtung diffus. Für den Brillenträger entsteht dadurch der Eindruck einer leuchtenden Fläche beispielsweise in Form eines leuchtenden Streifens, insbesondere einer leuchtenden Linie in Richtung der diffusen Abstrahlung. Vorzugsweise ist das Lichtfeld wesentlich breiter als die Pupille des Brillenträgers ausgebildet, d. h. zumindest 2 mal, 5 mal, 10 mal, und/oder 20 mal so breit wie die Pupille des Brillenträgers. Als besonders geeignet hat sich eine Breite von etwa 32 mm erwiesen. Somit kann der Brillenträger seine Position verlagern, ohne dass sich sein Seheindruck verändert, solange er sich im Lichtfeld des Fixationstargets befindet und das in der zweiten Ebene parallele Licht sieht. In anderen Worten "wandert" der sichtbare Streifen mit der Verlagerung des Brillenträgers "mit".

Aufgrund der Ausbildung des Lichtfeldes wird die Blickrichtung des Brillenträgers bei Betrachtung des Lichtfeldes durch die Richtung des Lichtfeldes vorgegeben, d. h. durch die Richtung der parallelen Strahlen. Ist beispielsweise die erste Ebene eine Vertikalebene im Bezugssystem der Erde und die zweite Ebene eine Horizontalebene im Bezugssystem der Erde, wird die Blickrichtung des Brillenträgers in horizontaler Richtung durch die Richtung des Lichts des Lichtfeldes vorgegeben. In vertikaler Richtung wird die Blickrichtung durch die vertikale Ausdehnung beschränkt. Somit kann der Brillenträger innerhalb des Lichtfeldes seine natürliche Blickhaltung einnehmen.

Zusätzlich zu den obigen Ausführungen wird der Brillenträger aufgrund der parallelen elektromagnetischen Strahlen bei Betrachtung des Lichtfeldes des Fixationstargets seinen Blick "ins Unendliche" richten. In anderen Worten empfindet der Brillenträger aufgrund der parallelen elektromagnetischen Strahlen des Lichtfeldes das Lichtfeld als "unendlich" entfernt. Somit nimmt der Brillenträger eine natürliche Kopf- und Körperhaltung ein, die einem natürlichen Sehen in die Ferne, insbesondere gerade aus in die Ferne entspricht. Vorteilhafterweise ist der Seheindruck des Brillenträgers von der genauen Position des Auges vor dem Fixationstarget, insbesondere vor dem Lichtfeld im Wesentlichen unabhängig, solange der Brillenträger die parallele elektromagnetische Strahlung betrachtet. Beispielsweise kann der Brillenträger seine Position in einer Richtung parallel zu der zweiten Ebene verlagern, beispielsweise in horizontaler Richtung, solange er die parallele elektromagnetische Strahlung des Lichtfeldes erblickt. In vertikaler Richtung ist der Brillenträger aufgrund der diffusen elektromagnetischen Strahlung frei in seiner Kopfbewegung, d. h. der Brillenträger kann beispielsweise den Kopf in der vertikalen Richtung frei bewegen, wenn beispielsweise die erste Ebene eine Vertikalebene ist, und seine natürliche Kopfhaltung einnehmen. Somit ist die Blickrichtung aufgrund der Richtung des parallelen Lichts lediglich in einer Raumrichtung vorgegeben, nämlich in der Horizontalrichtung. Ist das Lichtfeld breit, kann der Brillenträger den Kopf gegebenenfalls etwas drehen und/oder verlagern, wobei der sichtbare Streifen bei horizontaler Verlagerung des Kopfes "mitwandert". Ist das Lichtfeld schmal, ist der Brillenträger in seiner Kopfhaltung in horizontaler Richtung im Wesentlichen auf das schmale Lichtfeld beschränkt. In der beispielhaften vertikalen Richtung kann der Brillenträger seine Blickrichtung frei wählen. Dies kann gerade bei der Anpassung von Gleitsichtgläsern sehr vorteilhaft sein.

Ein Aspekt betrifft ein Herstellungsverfahren zum Herstellen des Fixationstargets gemäß dem voranstehenden Aspekt. Dabei kann es sich insbesondere um ein Ziehverfahren und/oder um einen Gießverfahren handeln, bei dem der transparente Linsenkörper z.B. aus Kunststoff hergestellt wird. Teil des Herstellungsverfahrens kann auch die Ausbildung eines Lichtliniengenerators sein, insbesondere gemäß einem der voranstehend beschriebenen Verfahren.

Ein Aspekt betrifft eine Zentriervorrichtung zum Bestimmen von optischen Zentrierparametern und/oder individuellen Parametern eines Brillenträgers mit einem Fixationstarget nach dem voranstehenden Aspekt.

Die Zentriervorrichtung kann zum Beispiel als die in der Druckschrift DE 10 2005 003 699 A1 offenbarte Zentriervorrichtung ausgebildet sein. Die Zentriervorrichtung kann zum Beispiel als ein Videozentriersystem ausgebildet sein. Die Zentriervorrichtung umfasst zumindest das Fixationstarget, eine Messeinrichtung, und eine Parameterberechnungseinrichtung. Die Zentriervorrichtung ist dazu ausgebildet und/oder konfiguriert, optische Zentrierparameter wie zum Beispiel eine Pupillendistanz, eine Einschleifhöhe einen Hornhautscheitelabstand und/oder individuelle Parameter wie z.B. einen Fassungsscheibenwinkel und/oder eine Vorneigung (jeweils in Gebrauchsstellung) zu bestimmen.

Dazu kann die Zentriervorrichtung eine Messeinrichtung aufweisen. Die Messeinrichtung kann zum Beispiel zumindest zwei Bildaufnahmeeinrichtungen zum Generieren von Bilddaten des Kopfes des Brillenträgers aus zumindest zwei Aufnahmerichtungen aufweisen. Die Bildaufnahmeeinrichtungen können zum Beispiel ein Stereobild des Kopfes des Brillenträgers generieren. Alternativ kann die Messeinrichtung auch lediglich eine Bildaufnahmeeinrichtung und/oder eine Beleuchtungseinrichtung wie zum Beispiel eine Musterprojektionseinrichtung aufweisen. Die Messeinrichtung kann insbesondere dazu ausgebildet sein, Bilddaten des Kopfes des Brillenträgers zu erzeugen. Die Bilddaten können den Kopf des Brillenträgers und eine Brillenfassung umfassen. Aus den Bilddaten kann die Messeinrichtung die Messposition des zumindest einen Auges des Brillenträgers ermitteln. Bevorzugt ermittelt die Messeinrichtung die Messpositionen beider Augen des Brillenträgers.

Die Zentriervorrichtung muss nicht zwingend als ein Zweikamerasystem ausgebildet sein, sondern kann auch als ein Mehrkamerasystem oder ein Einkamerasystem ausgebildet sein. Letzteres kann z.B. mit einem Aufsteckbügel zusammen verwendet werden.

Die Messeinrichtung kann weitere Elemente aufweisen, wie z.B. Spiegel, Linsen und/oder Gitter zum Umlenken der optischen Achsen der zumindest einen Bildaufnahmeeinrichtung. Weiterhin kann die Messeinrichtung z.B. Beleuchtungsmittel, einen Prozessor, einen Speicher und/oder eine Softwareimplementierung aufweisen.

Bei der Erzeugung der Bilddaten kann der Brillenträger in etwa an einem vorbestimmten Abstand vor der Zentriervorrichtung angeordnet sein. Bei der Erzeugung der Bilddaten nimmt der Brillenträger vorzugsweise seine Gebrauchsstellung ein, d.h. er trägt die Brillenfassung in einer möglichst natürlichen Haltung.

Um den Blick des Brillenträgers beim Ermitteln der Messposition(en) zu steuern und/oder auszurichten, wird vom Fixationstarget das Lichtfeld ausgesendet. Das Lichtfeld kann als ein im Wesentlichen rechteckiges Lichtfeld ausgebildet sein. Das Lichtfeld beleuchtet zumindest das eine Auge des Brillenträgers, bevorzugt zumindest beide Augen des Brillenträgers, besonders bevorzugt das gesamte Gesicht des Brillenträgers. Der Brillenträger kann angewiesen werden, dass vom Fixationstarget erzeugte Lichtfeld anzublicken. Dann beeinflusst und/oder steuert das Lichtfeld des Fixationstargets die Messposition und/oder die Messstellung des Auges.

Ein Aspekt betrifft die Verwendung eines Fixationstargets nach dem voranstehend beschriebenen Aspekt als Hilfe zum definierten Ausrichten der Blickrichtung und/oder zumindest eines Auges eines Brillenträgers, wobei mittels des Fixationstargets ein flächig ausgedehntes Lichtfeld erzeugt wird und der Brillenträger auf das Lichtfeld blickt. Dabei kann das Fixationstarget insbesondere in einer Zentriervorrichtung verwendet werden, um so optische Zentrierparameter und/oder Zentrierdaten des Brillenträgers bei einem entsprechend ausgerichteten Blick ermitteln zu können.

Ein Aspekt betrifft ein Verfahren zum Bestimmen von Zentrierparametern und/oder individuellen Parametern eines Brillenträgers, wobei mittels eines Fixationstargets gemäß dem voranstehend beschriebenen Aspekt die Blickrichtung und/oder zumindest ein Auge des Brillenträgers definiert ausgerichtet wird und die Zentrierparameter und/oder individuellen Parameter in dieser definiert ausgerichteten Position des Brillenträgers bestimmt werden.

Begriffe wie oben, unten, oberhalb, unterhalb, lateral, usw. beziehen sich - sofern nicht anders spezifiziert - auf das Bezugssystem der Erde in einer Betriebsposition des Gegenstands der Erfindung.

### KURZE BESCHREIBUNG DER ZEICHNUNGEN

Die Erfindung wird nachfolgend anhand von in Figuren gezeigten Ausführungsbeispielen näher beschrieben. Hierbei können gleiche oder ähnliche Bezugszeichen gleiche oder ähnliche Merkmale der Ausführungsformen kennzeichnen. Einzelne in den Figuren gezeigte Merkmale können in anderen Ausführungsbeispielen implementiert sein. Es zeigen:
- Fig. 1: in einer perspektivischen und schematischen Darstellung eine Zentriervorrichtung, welche einen Brillenträger vermisst;
- Fig. 2: in einer perspektivischen und schematischen Darstellung ein herkömmliches Fixationstarget einer Zentriervorrichtung;
- Fig. 3: in einer schematischen Draufsicht ein herkömmliches Fixationstarget einer Zentriervorrichtung;
- Fig. 4: einen Linsenkörper einer Ausführungsform eines Fixationstargets in einer schematischen, perspektivischen Darstellung;
- Fig. 5: den in Fig. 4 gezeigten Linsenkörper in einer schematischen Rückansicht;
- Fig 6: in einer schematischen Draufsicht den Strahlengang durch eine Ausführungsform eines Fixationstargets, bei welcher die Brennebene eines Linsenkörpers in einer planen Rückseite eines Linsenkörpers angeordnet ist;
- Fig. 7: in einer schematischen Draufsicht den Strahlengang durch eine zweite Ausführungsform eines Fixationstargets; bei welcher die Brennebene eines Linsenkörpers im Inneren dieses Linsenkörpers und beabstandet dessen sphärischer Rückseite angeordnet ist; und
- Fig. 8: in einer schematischen Draufsicht den Strahlengang durch eine dritte Ausführungsform eines Fixationstargets bei welcher die Brennebene eines Linsenkörpers im Inneren dieses Linsenkörpers und beabstandet dessen planer Rückseite angeordnet ist.

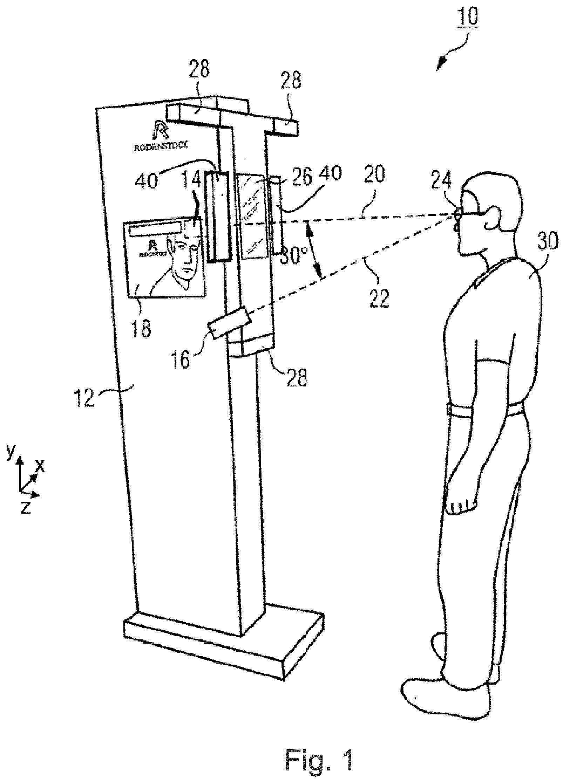

**Fig.1** zeigt eine schematische Perspektivenansicht einer Zentriervorrichtung 10 zum Bestimmen von optischen Zentrierparametern eines Brillenträgers 30. Die Zentriervorrichtung 10 weist eine Anordnungseinrichtung in Form eines Gehäuses und/oder einer Säule 12 auf, an welcher eine erste Bildaufnahmeeinrichtung 14 in Form einer oberen Kamera und eine zweite Bildaufnahmeeinrichtung 16 in Form einer seitlichen Kamera angeordnet ist. Ferner ist in die Säule 12 eine Datenausgabeeinrichtung in Form eines Monitors 18 integriert.

Die obere Kamera 14 befindet sich vorzugsweise im Inneren der Säule 12, beispielsweise wie in Fig. 1 gezeigt, zumindest teilweise auf gleicher Höhe wie der Monitor 18. In Betriebsstellung sind die obere Kamera 14 und die seitliche Kamera 16 derart angeordnet, dass sie Bilddaten des Kopfs des Brillenträgers 30 generieren können. Dabei können sich eine effektive optische Achse 20 der oberen Kamera 14 mit einer effektiven optischen Achse 22 der seitlichen Kamera 16 in einem Schnittpunkt 24 schneiden. Bei dem Schnittpunkt 24 der effektiven optischen Achsen 20, 22 handelt es sich vorzugsweise um den Punkt einer Nasenwurzel oder um den Mittelpunkt der Brücke.

Die obere Kamera 14 kann mittig hinter einem teildurchlässigen Spiegel 26 angeordnet sein. Die Bilddaten der oberen Kamera 14 werden durch den teildurchlässigen Spiegel 26 hindurch erzeugt. Die Bilddaten (im Folgenden Bilder genannt) der oberen Kamera 14 und der seitlichen Kamera 16 werden vorzugsweise an dem Monitor 18 ausgegeben.

Weiterhin können an der Säule 12 der Zentriervorrichtung 10 (z.B. drei) Leuchtmittel 28 angeordnet sein. Bei den Leuchtmitteln 28 kann es sich beispielsweise um Leuchtstäbe, wie Leuchtstoffröhren handeln. Die Leuchtmittel 28 können jedoch auch jeweils eine oder mehrere Glühbirnen, Halogenleuchten, Leuchtdioden, etc. aufweisen.

Die effektive optische Achse 20 der oberen Kamera 14 kann z.B. parallel zu der Nullblickrichtung des Brillenträgers 30 angeordnet sein. Die Nullblickrichtung entspricht der Fixierlinie der Augen des Brillenträgers 30 in Primärstellung. Die seitliche Kamera 16 kann derart angeordnet sein, dass die effektive optische Achse 22 der seitlichen Kamera 16 die effektive optische Achse 20 der oberen Kamera 14 in einem Schnittpunkt 24 unter einem Schnittwinkel von z.B. näherungsweise 30° schneidet. Bei dem Schnittpunkt 24 der effektiven optischen Achsen 20, 22 handelt es sich vorzugsweise um den Punkt einer Nasenwurzel des Brillenträgers 30. Hierbei sind auch andere Schnittwinkel möglich, z.B. kann der Schnittwinkel kleiner als etwa 60° ausgebildet sein. Es ist nicht notwendig, dass sich die effektiven optischen Achsen 20, 22 schneiden.

Die Kameras 14, 16 können ausgelegt sein, jeweils einzelne Bilder eines Teilbereichs des Kopfes des Brillenträgers 30 zu erzeugen. Es ist auch möglich, dass mittels der Kameras 14, 16 Videosequenzen aufgenommen werden und diese Videosequenzen zur weiteren Auswertung benutzt werden. Die Bilddaten und/oder Bilder können zur weiteren Auswertung zeitsynchronisiert aufgenommen werden.

In Betriebsstellung kann der Brillenträger 30 derart angeordnet und/oder positioniert sein, dass sein Blick auf den teildurchlässigen Spiegel 26 gerichtet ist, wobei der Benutzer etwa auf die Abbildung seiner Nasenwurzel in dem Spiegelbild des teildurchlässigen Spiegels 26 blickt.

Die Bildaufnahmeeinrichtungen 14, 16 können Elemente einer Messeinrichtung der Zentriervorrichtung 10 sein. Weitere Elemente der Messeinrichtung können z.B. im Inneren des Gehäuses 12 angeordnet sein, wie z.B. ein Prozessor, ein Speicher und/oder eine Software. Mittels der von den Bildaufnahmeeinrichtungen 14, 16 aufgenommenen Bilddaten kann die Messeinrichtung die Messposition zumindest eines Auges der Brillenträgers 30 ermitteln, bevorzugt die Messpositionen beider Augen der Brillenträgers 30.

Die Zentriervorrichtung 10 weist ferner zumindest ein Fixationstarget 40 auf. Dabei kann die Zentriervorrichtung 10 auch zwei Fixationstargets 40 aufweisen, z.B. zum Ausrichten des Brillenträgers 30 in unterschiedlichen Positionen.

Die Messeinrichtung nimmt bevorzugt genau dann Bilddaten des Brillenträgers 30 auf, wenn dieser ein vom Fixationstarget 40 kontrolliert ausgesendetes Lichtfeld fixiert.

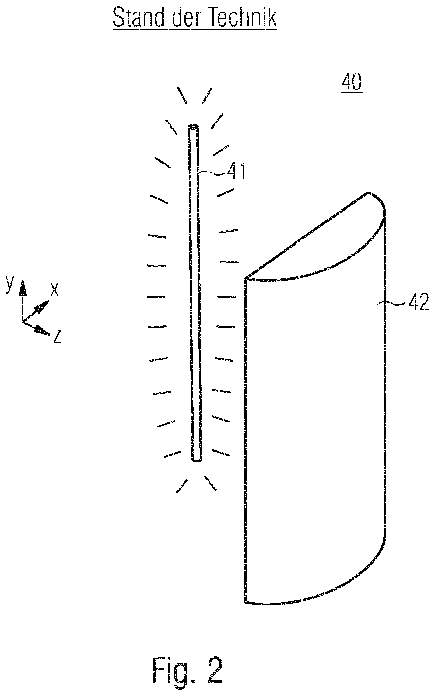

**Fig. 2** zeigt eine schematische, perspektivische Ansicht eines herkömmlichen Fixationstargets 40. Das Fixationstarget 40 weist eine Zylinderlinse 42 sowie eine Lichtquelle 41 auf. Die Lichtquelle 41 kann beispielsweise eine LED, insbesondere eine homogene LED, eine Glühlampe und/oder eine ähnliche aktive Lichtquelle umfassen. Die Lichtquelle 41 kann etwa an einer Brennlinie der Zylinderlinse 42 angeordnet sein.

In der gezeigten Ausführungsform ist die Lichtquelle 41 etwa stabförmig und/oder zylinderförmig ausgebildet. Die Lichtquelle 41 kann somit im Wesentlichen als eine leuchtende Linie ausgebildet sein. Die stabförmige Lichtquelle 41 ist etwa vertikal angeordnet, d.h. dass die Zylinderachse der Lichtquelle 41 etwa vertikal angeordnet ist. Die Vertikalrichtung ist in den Figuren als y-Richtung eines kartesischen Koordinatensystems gekennzeichnet.

Die Zylinderachse der Zylinderlinse 42 ist ebenfalls etwa vertikal, d.h. in y-Richtung, angeordnet. Die Lichtquelle 41 ist in negative z-Richtung von der Zylinderlinse 42 beabstandet. Dabei ist die z-Richtung eine etwa horizontal ausgerichtete Richtung, welche etwa senkrecht von der konvexen Seite der Zylinderlinse 42 weg weist in Richtung des von der Lichtquelle 41 durch die Zylinderlinse 42 abgestrahlten Lichtfelds.

**Fig. 3** zeigt in einer schematischen Draufsicht das herkömmliche Fixationstarget 40. Dabei ist gezeigt, wie das von der Lichtquelle 41 abgestrahlte Licht die etwa flache Rückseite der Zylinderlinse 42 beleuchtet. Dieses Licht dringt in die Zylinderlinse 42 ein und wird von der Zylinderlinse 42 an ihrer konvexen Seite, welche der Lichtquelle 41 abgewandt ist, als etwa parallel ausgerichtete Lichtstrahlen 50 abgestrahlt. Die Lichtstrahlen 50 bilden das vom Fixationstarget 40 tatsächlich erzeugte und/oder abgestrahlte Lichtfeld aus.

Die Lichtstrahlen 50 sind innerhalb der x-z-Ebene etwa parallel zueinander ausgerichtet und strahlen in etwa in z-Richtung, d.h. etwa horizontal vom Fixationstarget 40 (und der Zentriervorrichtung 10) weg in Richtung zum Brillenträger 30 hin (vgl. auch das in Fig. 1 gezeigte Koordinatensystem). Die z-Richtung fällt somit mit der optischen Achse des Fixationstargets zusammen. In y-Richtung sind die Lichtstrahlen 50 diffus.

Idealerweise ist die Lichtquelle 41 exakt in der Brennlinie der Zylinderlinse 42 angeordnet. Dann ist die elektromagnetische Strahlung, welche von den Lichtstrahlen 50 bereitgestellt wird, exakt parallel. Sofern die Zylinderachse und Brennlinie der Zylinderlinse 42 exakt vertikal angeordnet sind, breiten sich die Lichtstrahlen 50 exakt in einer horizontalen Ebene im Bezugssystem der Erde aus.

Eine solche x-z-Ebene ist z.B. in Fig. 3 gezeigt.

Die x-Richtung des benutzten Koordinatensystems ist ebenfalls etwa horizontal angeordnet, steht senkrecht auf der y- und der z-Richtung, und weist in einer seitlichen Richtung von dem Fixationstarget 40 weg (vgl. Fig. 2). Die x-Richtung kann z.B. etwa parallel zur flachen, zur Brennlinie hin ausgerichteten Rückseite der Zylinderlinse 42 angeordnet sein und/oder in eine laterale Richtung weisen.

Eine optische Achse des Fixationstargets 40 ist eine Achse, die im Wesentlichen parallel zu der elektromagnetischen Strahlung der Lichtstrahlen 50 ausgerichtet ist. Die optische Achse des Fixationstargets weist somit in z-Richtung.

Das Lichtfeld des Fixationstargets 40 wird von der vertikalen, diffus leuchtenden Lichtquelle 41 und der vertikal orientierten Zylinderlinse 42 gebildet. Hierbei ist die diffus leuchtende Lichtquelle 41 in der Brennlinie angeordnet, welche in der Zylinderachse der konvexen und zylindrischen Vorderfläche der Zylinderlinse 42 angeordnet ist. Das resultierende Lichtfeld (vgl. Fig. 3) ist entlang der x-Richtung in der horizontalen Ebene (x-z-Ebene) parallel zur Achse des Fixationstargets 40, also der z-Richtung, und diffus in der vertikalen y-Richtung ausgebildet. Dadurch wird ein Auge im Bereich des Lichtfeldes bei dessen Betrachtung horizontal parallel zur Achse des Fixationstargets ausgelenkt, aber vertikal nicht beeinflusst.

Hierbei muss die Brennlinie nicht (wie in den Figuren 2 und 3 gezeigt) außerhalb des Linsenelementes der Zylinderlinse 42 liegen. In einer monolithischen Bauform kann die Brennlinie und damit die leuchtende Linie auch an der Rückfläche oder innerhalb des Linsenelementes liegen (vgl. nachfolgende Figuren).

Wird dieses Fixationstarget 40 in der Zentriervorrichtung 10 und/oder einem Videozentriersystem eingesetzt, kann die Achse des Fixationstargets (z-Achse) parallel zur Achse der Zentriervorrichtung 10 ausgerichtet werden. Diese Achse der Zentriervorrichtung 10 kann beispielsweise die effektive optische Achse einer der Bildaufnahmeeinrichtungen sein, z.B. die erste effektive optische Achse 20 der ersten Bildaufnahmeeinrichtung 14. Allgemein kann die Achse des Fixationstargets 40 parallel zur effektiven optischen Achse einer Zentriervorrichtung mit nur einer Kamera, parallel zu einer primären Kamera einer Zentriervorrichtung mit zwei oder mehreren Kameras, oder einer Symmetrieachse bei einer Zentriervorrichtung mit mehreren seitlich zueinander angeordneten Kameras angeordnet werden. Bei einer Zentriervorrichtung, welche einen Spiegel 26 aufweist, in dem sich der Brillenträger 30 beobachten kann, kann die Achse der Zentriervorrichtung auch in Abhängigkeit der Ausrichtung der Spiegelfläche (typischerweise als Normale auf die Spiegelfläche) definiert sein.

Um eine Fehlauslenkung der Augen des Brillenträgers 30 zu vermeiden, sollte die horizontale Komponente der Richtung des Lichtfelds über den ganzen Bereich gleichmäßig parallel und parallel zur Achse der Zentriervorrichtung 10 angeordnet sein. Anderenfalls würde das Auge in der Horizontalen abweichend von dieser Soll-Richtung ausgelenkt, nämlich in der jeweiligen lokalen Richtung des Lichtfeldes am Ort der Pupille des Brillenträgers 30.

Um ein derartiges Lichtfeld zur Verfügung zu stellen, müssen bei herkömmlichen Fixationstargets zwei Bedingungen erfüllt werden: Erstens darf die Zylinderlinse 42 nahezu keine Abbildungsfehler aufweisen, was hohe Ansprüche an deren Fertigung stellt. Zweitens muss die diffus leuchtende Lichtquelle 41 genau in der Brennlinie der Zylinderlinse 42 stehen. Dies erfordert ein justierbares System und/oder passgenaue Komponenten. Das justierbare System bedarf einer komplexen Optik und Mechanik, die eine Justage der beiden optischen Elemente 41, 42 zueinander sowie zur Achse der Zentriervorrichtung 10 ermöglichen. Weiterhin bedarf es hierfür einer aufwändigen Justage während und/oder nach der Fertigung. Die passgenauen Komponenten sollten derart geringe Toleranzen und entsprechende Passungen aufweisen, dass beim Zusammenfügen der Bauteile auf Grund der Toleranzkette zwangsläufig ein Lichtfeld mit der notwendigen Qualität entsteht. Dies stellt besonders hohe Anforderungen an die Fertigung der Zylinderlinse 42. Die dazu notwendigen Toleranzen hinsichtlich der lateralen und axialen Lage der Brennlinie bezogen auf Flächenelemente der Zylinderlinse 42 können mit kostengünstigen Standardprozessen nicht erreicht werden.

Dies gilt insbesondere für Lichtfelder mit großer lateraler Ausdehnung (insbesondere in x-Richtung), da sich hierbei Abbildungsfehler einer Linse (insbesondere bei einfachen Zylinderlinsen mit sphärischem Schnitt) besonders deutlich auswirken. Es erfordert schon aufwändig herzustellende Linsen mit insbesondere asphärischen Schnitten, um die Abbildungsfehler zu vermeiden.

Eine Aufgabe der Erfindung kann es sein, Vorrichtungen und Verfahren bereitzustellen, die eine hohe Genauigkeit bei der Messung von Parametern, insbesondere von optischen Zentrierparametern, des Auges und/oder des Systems Brille-Auge mit weniger anspruchsvollen Komponenten und/oder Systemen zu geringen Herstellungskosten ermöglicht. Beispiele für derartige Parameter sind die bekannten Zentrier- und Individualparameter sowie die Augendrehpunktlage, Position, Form und Größe der Pupille sowie die Position des Hornhautscheitels.

Um die voranstehend aufgeführten Kosten zu reduzieren, kann das herkömmliche Fixationstarget 40 ersetzt werden durch das nachfolgend beschriebene, erfindungsgemäße Fixationstarget 70.

**Fig. 4** zeigt einen Linsenkörper 60 einer Ausführungsform eines Fixationstargets 70 (vgl. Fig. 6) in einer schematischen, perspektivischen Darstellung. Der Linsenkörper 60 ist monolithisch ausgebildet und erstreckt sich in Abstrahlrichtung A, welche in Betriebsposition etwa oder genau parallel zur z-Richtung ausgerichtet sein kann (vgl. Fig. 1), von seiner flachen Rückseite 62 bis zu seiner konvexen Abstrahlseite 61.

Die Abstrahlseite 61 kann sphärisch oder asphärisch ausgebildet sein. Sie kann die Form einer in y-Richtung, also z.B. vertikal, ausgerichteten Zylinderlinse aufweisen. Die Abstrahlseite 61 kann ähnlich geformt sein wie die Abstrahlseite der in den Figuren 2 und 3 gezeigten der Zylinderlinse 42. Anders als die dort gezeigte Zylinderlinse 42 ist der Linsenkörper 60 jedoch entgegen der Abstrahlrichtung A verlängert ausgebildet bis hin zu seiner flachen Rückseite 62. Die Brennlinie 69 ist allerdings etwas anders relativ zur Abstrahlseite 61 angeordnet als bei der vorbekannten Zylinderlinse 42, da beim Linsenkörper 60 zwischen Brennlinie 69 und Abstrahlseite 61 keine Brechung an der Rückseite 62 erfolgt.

Die Rückseite 62 kann in Betriebsposition in einer Vertikalebene ausgerichtet sein, welche etwa senkrecht zur Abstrahlrichtung A angeordnet ist, z.B. etwa parallel zu einer x-y-Ebene. Die Abstrahlseite 61 ist so geformt, dass sie eine Zylinderlinse ausbildet, deren Brennlinie 69 genau entlang der Rückseite 62 verläuft. Die Brennlinie 69 ist in Fig. 4 als gestrichelte Linie gezeigt. Die Brennlinie 69 liegt innerhalb der Brennebene des Linsenkörpers 60.

Der Linsenkörper 60 ist aus einem transparenten Material ausgebildet, z.B. aus Glas und/oder Kunststoff. Das Material des Linsenkörpers 60 dient zur Bereitstellung von Außenflächen zur optischen Formung von Licht bei Aus- und/oder Eintritt aus dem und/oder in den Linsenkörper 60.

Der Linsenkörper 60 kann weiterhin zwei einander gegenüberliegende, laterale Seitenflächen 63 aufweisen, welche jeweils etwa senkrecht zur Rückseite 62 angeordnet sein können. Die Abstrahlfläche 61, die Rückseite 62, und ggf. die Seitenflächen 63 können den Linsenkörper 60 in sämtliche horizontale Richtungen begrenzen. Zudem kann der Linsenkörper 60 eine Oberseite und eine Unterseite aufweisen, welche den Linsenkörper 60 nach oben und unten begrenzen können. Die Rückseite 62, die Seitenflächen 63, die Oberseite und/oder die Unterseite können jeweils als eine ebene Fläche ausgebildet sein, während die Abstrahlseite 61 konkav und/oder konvex ausgebildet sein kann.

Die Rückseite 62 weist entlang der Brennlinie 69 einen Lichtgenerator auf, welcher als Lichtliniengenerator 65 ausgebildet ist und zur Beugung und/oder Brechung von in etwa in Abstrahlrichtung auf die Rückseite 62 einfallendem Licht dient.

**Fig. 5** zeigt einen Ausschnitt des Linsenkörpers 60 in einer schematischen Rückansicht auf die Rückseite 62. Die Rückseite 62 ist fast vollständig geschwärzt ausgebildet, da sie mit einer Schwärzung 66 versehen ist, welche in Fig. 5 schraffiert gezeigt ist. Lediglich ein schmaler Spalt entlang der Brennlinie 69 ist ungeschwärzt ausgebildet und somit transparent belassen. Genau dieser ungeschwärzte Spalt stellt den Lichtliniengenerator 65 bereit, der ebenfalls in der Brennlinie 69 ausgebildet und angeordnet ist.

Die Schwärzung 66 kann z.B. als Fotolack implementiert sein. Dieser kann von der Abstrahlseite 61 her mittels eines rückwärts gerichteten Solllichtfelds, z.B. mit rückwärts gerichteten parallelen Lichtstrahlen 50 (vgl. Fig. 3 und 6) so beleuchtet werden, dass nur die Brennlinie 69 beleuchtet wird. Wird als Schwärzung Positivlack verwendet, bildet dieser nach einer Entwicklung übrigbleibende Lack die Schwärzung 66 aus, während der Spalt des Lichtliniengenerators 65 freibleibt. Dadurch kann sichergestellt werden, dass der freigelassene Spalt genau auf der Rückseite 62 entlang der Brennlinie 69 ausgebildet wird.

Alternativ oder zusätzlich zur Schwärzung 66 kann die Rückseite 62 (z.B. bis auf den Spalt) auch aufgeraut ausgebildet sein.

Zusätzlich können z.B. die Seitenflächen 63 und/oder die Ober- und Unterseite geschwärzt und/oder aufgeraut ausgebildet sein, um Streulicht zu reduzieren.

**Fig 6** zeigt in einer schematischen Draufsicht den Strahlengang durch eine Ausführungsform eines Fixationstargets 70. Das Fixationstarget 70 weist den Linsenkörper 60 sowie eine Lichtquelle 71 auf, welche in der Nähe der Rückseite 62 angeordnet ist. Die Lichtquelle 71 muss kein diffuses Licht ausstrahlen. Die Lichtquelle 71 kann als eine gewöhnliche (aktive) Lichtquelle ausgebildet sein, z.B. als eine LED.

Die Lichtquelle 71 beleuchtet die Rückseite 62 des Linsenkörpers 60, wobei die Schwärzung 66 ein Eindringen des Lichts in den Linsenkörper 60 überall bis auf entlang des ungeschwärzt ausgebildeten Spalts reduziert und/oder verhindert.

Die "Schwärzung" 66 bedeutet hierbei nicht zwingend, dass die Rückseite 62 tatsächlich "schwarz" gefärbt sein muss. Sie kann auch mit einer anderen Farbe, bevorzugt einer dunklen Farbe, gefärbt sein, von einem Lack bedeckt und/oder aufgeraut sein. Der Spalt in der Schwärzung 66 kann z.B. mittels eines normalen Lacks und/oder einer Farbe erzeugt werden, wobei der Spalt vor dem Auftragen des Lacks und/oder der Farbe abgeklebt wird. Nach dem Trocknen des Lacks und/oder der Farbe kann die Abdeckung abgenommen werden und dadurch der freie Spalt ausgebildet werden. Die Abdeckung kann auch durch Photolack bereitgestellt werden. Dabei kann eine Belichtung der zu schwätzenden (bei Verwendung von Negativ-Lack) bzw. nicht zu schwärzenden Bereiche (bei Verwendung von PositivLack) erfolgen. Ähnliches gilt bei Ausbildung einer Aufrauhung an Stelle der Schwärzung 66.

Der Spalt des Lichtliniengenerators 65 wirkt als spaltförmige Blende für das Licht der Lichtquelle 71 und generiert somit in der Brennlinie 69 diffuses Licht, welches aus der Abstrahlseite 61 als parallele Lichtstrahlen 50 austritt. Die Lichtstrahlen 50 sind in einer horizontalen Richtung parallel zueinander und strahlen in Abstrahlrichtung A.

Diese Lichtstrahlen 50 bilden das Lichtfeld des Fixationstargets 70 aus. Es entsteht durch die Zylinderlinse des Linsenkörpers 60 das Lichtfeld, welches als Fixationstarget z.B. in einer Zentriervorrichtung 10 (vgl. Fig. 1) genutzt werden kann. Die Schwärzung 66 oder Aufrauhung der Rückseite 62 reduziert dabei Mehrfachreflektionen. Mehrfachreflektionen können weiter reduziert werden durch zusätzliche Schwärzung oder Aufrauhung der lateralen Seitenflächen des Linsenkörpers 60.

In einer alternativen, nicht in den Figuren gezeigten Ausführungsform eines Linsenkörpers ist die Brennlinie im Volumen des Linsenkörpers angeordnet. Bei dieser Bauform kann die leuchtende Linie durch eine Streuung an Streuzentren erzeugt werden, welche exakt an der Position der Brennlinie ausgebildet sind. Die Baulänge des Linsenkörpers entgegen der Abstrahlrichtung ist dabei (im Gegensatz zu der in den Figuren 4-6 gezeigten Ausführungsform) etwas länger die Brennweite.

Die Streuzentren können beispielsweise mit einem Laserschreibverfahren in das Material des Linsenkörpers eingebracht werden, ähnlich zu einer Glasinnengravur.

Eine Beleuchtung der Streuzentren zu deren Ausbildung kann mittels einer Beleuchtungsquelle erfolgen, deren Licht an einer (z.B. noch) nicht aufgerauten Seite des Linsenkörpers eingekoppelt wird. Dies kann an der Oberseite und/oder Unterseite und/oder an einer oder beiden Seitenflächen und/oder durch die Abstrahlfläche erfolgen.

Um sicherzustellen, dass die Streuzentren im Volumen des Linsenkörpers exakt in der Brennlinie der vorderseitigen Abstrahlseite liegen, kann die Erzeugung mit Hilfe eines Lichtbündels mit der gewünschten Eigenschaft erfolgen, also z.B. in sich parallele Strahlung, welche auch parallel zur Achse des Fixationstargets ausgerichtet ist. Diese Strahlung kann durch die Abstrahlseite eingestrahlt werden und dadurch an den entsprechenden Punkten entlang der Brennlinie fokussiert werden.

Das Fixationstarget ermöglicht die Erzeugung eines Lichtfeldes durch ein einzelnes Bauelement, welches gleichzeitig die Funktion einer Zylinderlinse, eines Tubus und einer (passiven) leuchtenden Linie eines Fixationstargets übernimmt.

Dadurch wird eine Reduktion der Anzahl der für ein Fixationstarget benötigten Bauteile mit einer entsprechenden Kostensenkung ermöglicht.

Der Wegfall einer Grenzfläche zwischen Zylinderlinse 42 und Lichtquelle 41 (vgl. Figuren 2 und 3) kann je nach gewählter Geometrie Mehrfachreflexionen verhindern, was einen Verzicht auf eine Antireflexbeschichtung an dieser Grenzfläche erlaubt.

Da eine Justage der Lichtquelle relativ zum Linsenkörper überflüssig wird zur Erzeugung des korrekt ausgerichteten Lichtfeldes, wird eine sehr schnelle und kosteneffiziente Justage und Herstellung des Fixationstargets ermöglicht.

Die Stabilität des Fixationstargets 70 kann dadurch verbessert sein, dass es weniger Einzelteile aufweist und diese dadurch weniger anfällig für eine Dejustage sein können.

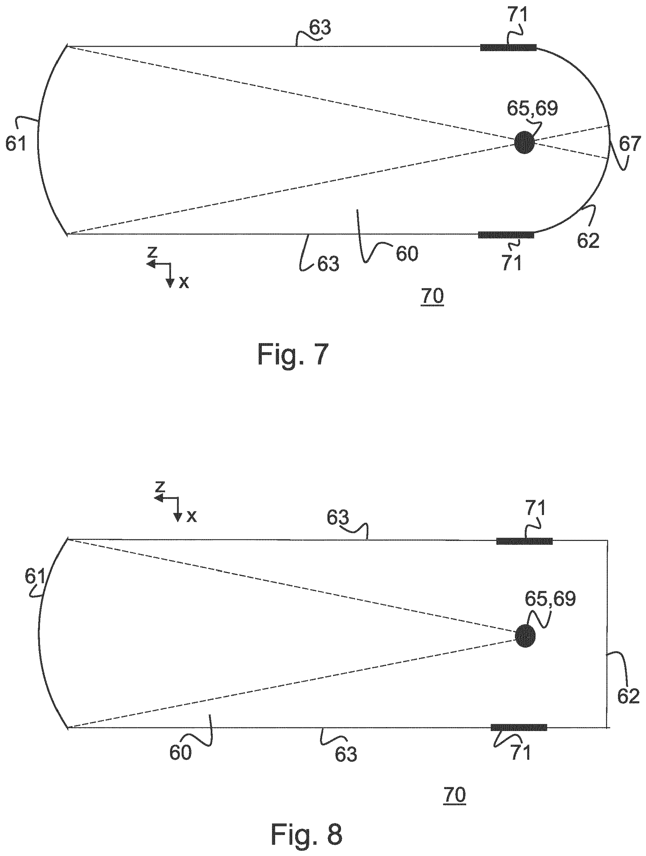

**Fig. 7** zeigt in einer schematischen Draufsicht den Strahlengang durch eine zweite Ausführungsform eines Fixationstargets 70. Hier ist der Lichtliniengenerator 65 im Inneren des Linsenkörpers 60 ausgebildet als ein passives Bauelement, also z.B. als aneinandergereihte Streuzentren und/oder als phosphoreszierender Farbstoff entlang der Brennlinie 69. Der Linsenkörper 60 erstreckt sich entgegen der z-Richtung von der sphärischen oder asphärischen Abstrahlseite 61 bis zur konvexen, z.B. sphärischen oder asphärischen Rückseite 62.

Der Lichtliniengenerator 65 wird von zwei an den Seitenflächen 63 angeordneten Lichtquellen 71 angeleuchtet, wodurch der Lichtliniengenerator 65 zum Bereitstellen der Lichtstrahlen 50 angeregt wird (vgl. auch Fig. 6). Die Lichtquellen 71 können z.B. als flache LEDs ausgebildet sein und sind angrenzend und/oder anliegend an die Seitenflächen 63 angeordnet. Dabei können die Lichtquellen 71 z.B. in der Brennebene angeordnet sein, welche entlang der Brennlinie 69 in der x-y-Ebene angeordnet ist.

An der Rückseite 62 kann eine Verspiegelung 67 angeordnet sein, um gestreutes Licht in den Lichtliniengenerator 65 einkoppeln zu können. Die Verspiegelung kann sich dabei in y-Richtung von einem z.B. oberen zu einem z.B. unteren Ende des Linsenkörpers 60 erstrecken und/oder zumindest einen mittigen Bereich der Rückseite 62 bedecken, welcher der Abstrahlseite 61 bezüglich der Brennlinie 69 gegenüberliegt (in Fig. 7 durch gerade, gestrichelte Linien gekennzeichnet). Weitere Außenflächen des Linsenkörpers 60, also z.B. die Seitenflächen 63, die neben der Verspiegelung 67 verbleibenden Bereiche der Rückseite 62 und/oder Kanten der Abstrahlseite 61 können zumindest bereichsweise entspiegelt, geschwärzt und/oder aufgeraut ausgebildet sein. Die Abstrahlseite 61 ist bevorzugt (z.B. vollständig) entspiegelt ausgebildet.

**Fig. 8** zeigt in einer schematischen Draufsicht den Strahlengang durch eine dritte Ausführungsform eines Fixationstargets 70, bei welcher der Lichtliniengenerator 65 ebenfalls im Inneren des Linsenkörpers 60 ausgebildet ist als ein passives Bauelement, also z.B. als aneinandergereihte Streuzentren und/oder als phosphoreszierender Farbstoff entlang der Brennlinie 69. Der Linsenkörper 60 erstreckt sich entgegen der z-Richtung von der sphärischen oder asphärischen Abstrahlseite 61 bis zur planen Rückseite 62.

Ähnlich wie bei der in Fig. 7 gezeigten Ausführungsform wird der Lichtliniengenerator 65 von zwei an den Seitenflächen 63 angeordneten Lichtquellen 71 angeleuchtet, wodurch der Lichtliniengenerator 65 zum Bereitstellen der Lichtstrahlen 50 angeregt wird (vgl. auch Fig. 6). Die Lichtquellen 71 können z.B. als flache LEDs ausgebildet sein und sind angrenzend und/oder anliegend an die Seitenflächen 63 angeordnet. Dabei können die Lichtquellen 71 z.B. in der Brennebene angeordnet sein, welche entlang der Brennlinie 69 in der x-y-Ebene angeordnet ist.

Außenflächen des Linsenkörpers 60, also z.B. die Seitenflächen 63 beidseits neben den Lichtquellen 71, die Rückseite 62 und/oder Kanten der Abstrahlseite 61 können zumindest bereichsweise entspiegelt, geschwärzt und/oder aufgeraut ausgebildet sein. Die Abstrahlseite 61 ist wiederum bevorzugt (z.B. vollständig) entspiegelt ausgebildet.

### Bezugszeichenliste

- 10: Zentriervorrichtung
- 12: Gehäuse
- 14: erste Bildaufnahmeeinrichtung
- 16: zweite Bildaufnahmeeinrichtung
- 18: Monitor
- 20: erste effektive optische Achse
- 22: zweite effektive optische Achse
- 24: Schnittpunkt
- 26: Spiegel
- 28: Leuchtmittel

- 30: Brillenträger

- 40: Fixationstarget
- 41: Lichtquelle
- 42: Zylinderlinse

- 50: Lichtstrahlen

- 60: Linsenkörper
- 61: Abstrahlseite
- 62: Rückseite
- 63: Seitenfläche
- 65: Lichtliniengenerator
- 66: Schwärzung
- 67: Verspiegelung
- 69: Brennlinie

- 70: Fixationstarget
- 71: Lichtquelle

- A: Abstrahlrichtung

## Patentansprüche

1. Fixationstarget (70) zum Erzeugen eines Lichtfelds zum Ausrichten der Blickrichtung eines Brillenträgers (30) bei Vermessung des Brillenträgers (30) mittels einer Zentriervorrichtung (10), mit:
- einem transparenten Linsenkörper (60), mittels welchem im Betrieb eine elektromagnetische Strahlung des erzeugten Lichtfelds optisch geformt wird;
- einer optischen Abstrahlseite (61) des Linsenkörpers (60), aus welcher im Betrieb die elektromagnetische Strahlung des erzeugten Lichtfelds in eine Abstrahlrichtung (A) austritt; und
- einer Rückseite (62) des Linsenkörpers (60) an einer der Abstrahlseite (61) abgewandten Rückseitenfläche des Linsenkörpers (60);
wobei:
- entlang zumindest eines Abschnitts der Brennebene am und/oder im Linsenkörper (60) ein Lichtgenerator (65) ausgebildet ist, von welchem im Betrieb eine elektromagnetische Strahlung in Richtung zur optischen Abstrahlseite (61) hin derart abgestrahlt wird, dass diese elektromagnetische Strahlung nach dem Austritt aus der Abstrahlseite (61) das vom Fixationstarget (70) erzeugte Lichtfeld ausbildet;
- das Fixationstarget (70) eine Lichtquelle (71) aufweist zur Beleuchtung des Lichtgenerators (65) derart, dass der Lichtgenerator (65) eine von der Lichtquelle (71) emittierte elektromagnetische Strahlung formt und als elektromagnetische Strahlung in Richtung zur optischen Abstrahlseite (61) hin derart abstrahlt, dass diese elektromagnetische Strahlung nach dem Austritt aus der Abstrahlseite (61) das vom Fixationstarget erzeugte Lichtfeld derart ausbildet, dass das Lichtfeld in einer horizontalen Ebene parallel zur Ausbreitungsrichtung und diffus in einer vertikalen Richtung ausgebildet ist; und
- sich der transparente Linsenkörper (60) von der Abstrahlseite (61) entgegen der Abstrahlrichtung (A) bis zur Rückseite (62) und dabei bis zumindest zu einer Brennebene des Linsenkörpers (60) erstreckt.

2. Fixationstarget (70) nach Anspruch 1, wobei der Lichtgenerator (65) als ein passiver Lichtgenerator (65) ausgebildet ist, welcher nur unter Beleuchtung durch die Lichtquelle (71) die elektromagnetische Strahlung des Lichtfelds bereitstellt.

3. Fixationstarget (70) nach einem der vorangegangenen Ansprüche, wobei die Brennebene zumindest abschnittsweise genau an der Rückseite (62) des Linsenkörpers (60) angeordnet ist.

4. Fixationstarget (70) nach einem der Ansprüche 1 und/oder 2 und nach Anspruch 3, wobei der Lichtgenerator (65) dadurch bereitgestellt ist, dass die Rückseite (62) des Linsenkörpers (60) bis auf zumindest einen Abschnitt und/oder Bereich entlang der Brennebene geschwärzt und/oder aufgeraut ausgebildet ist.

5. Fixationstarget (70) nach einem der Ansprüche 1 und 2, wobei die Brennebene zumindest abschnittsweise im Inneren des Linsenkörpers angeordnet ist.

6. Fixationstarget (70) nach einem der Ansprüche 1 und/oder 2 und nach Anspruch 5, wobei der Lichtgenerator dadurch bereitgestellt ist, dass im Inneren des Linsenkörpers entlang zumindest eines Abschnitts der Brennebene Streuzentren ausgebildet sind.

7. Fixationstarget (70) nach einem der vorangegangenen Ansprüche, wobei zumindest eine Außenfläche (61, 62, 63) des Linsenkörpers (60) zumindest teilweise geschwärzt und/oder aufgeraut ausgebildet ist.

8. Fixationstarget (70) nach einem der vorangegangenen Ansprüche, wobei zumindest eine Außenfläche (61, 62, 63) des Linsenkörpers (60) mit einer Antireflexbeschichtung beschichtet ist.

9. Fixationstarget (70) nach einem der vorangegangenen Ansprüche, wobei der transparente Linsenkörper (60):
- als eine sphärische oder asphärische Zylinderlinse ausgebildet ist mit einer Brennlinie (69), welche in der Brennebene angeordnet ist.

10. Fixationstarget (70) nach den Ansprüchen 1 und 9, wobei der Lichtgenerator als ein Lichtliniengenerator (65) ausgebildet ist, welcher etwa entlang zumindest eines Abschnitts der Brennlinie (69) der Zylinderlinse angeordnet ist.

11. Fixationstarget (70) nach einem der vorangegangenen Ansprüche, wobei der transparente Linsenkörper (60) aus Kunststoff gegossen und/oder gezogen ist.

12. Fixationstarget (70) nach einem der vorangegangenen Ansprüche, wobei das Fixationstarget (70) derart ausgebildet ist, dass
- die elektromagnetische Strahlung des Lichtfelds in einer ersten vorbestimmbaren Ebene im Wesentlichen diffus ausgebildet ist und
- die elektromagnetische Strahlung des Lichtfelds in einer zweiten vorbestimmbaren Ebene, die etwa senkrecht zu der ersten Ebene angeordnet ist, im Wesentlichen parallel ausgebildet ist.

13. Zentriervorrichtung (10) zum Bestimmen von optischen Zentrierparametern und/oder individuellen Parametern eines Brillenträgers (30) mit einem Fixationstarget (70) nach einem der vorangehenden Ansprüche.

14. Verwendung eines Fixationstargets (70) nach einem der der Ansprüche 1 bis 12 als Hilfe zum definierten Ausrichten der Blickrichtung und/oder zumindest eines Auges eines Brillenträgers (30), wobei mittels des Fixationstargets (70) ein flächig ausgedehntes Lichtfeld erzeugt wird und der Brillenträger (30) auf das Lichtfeld blickt.

15. Verfahren zum Bestimmen von Zentrierparametern und/oder individuellen Parametern eines Brillenträgers (30), wobei mittels eines Fixationstargets (70) nach einem der der Ansprüche 1 bis 12 die Blickrichtung und/oder zumindest ein Auge des Brillenträgers (30) definiert ausgerichtet wird und die Zentrierparameter und/oder individuellen Parameter in dieser definiert ausgerichteten Position des Brillenträgers (30) bestimmt werden.

## Claims

1. A fixation target (70) for generating a light field to align the line of sight of a spectacle wearer (30) during the examination of the spectacle wearer (30) using a centering device (10), comprising:
- a transparent lens body (60), by means of which, during operation, electromagnetic radiation of the generated light field is optically shaped;
- an optical emission side (61) of the lens body (60), from which, during operation, the electromagnetic radiation of the generated light field emerges in an emission direction (A); and
- a rear side (62) of the lens body (60) on a rear surface of the lens body (60) facing away from the emission side (61);
wherein:
- a light generator is formed along at least a portion of the focal plane on and/or within the lens body (60) (65) is formed, from which, during operation, electromagnetic radiation is emitted toward the optical emission side (61) in such a way that this electromagnetic radiation, after exiting the emission side (61), forms the light field generated by the fixation target (70);
- the fixation target (70) comprises a light source (71) for illuminating the light generator (65) such that the light generator (65) shapes the electromagnetic radiation emitted by the light source (71) and emits it as electromagnetic radiation toward the optical emission side (61) such that, after exiting the emission side (61), this electromagnetic radiation forms the light field generated by the fixation target in such a way that the light field is formed in a horizontal plane parallel to the direction of propagation and is diffuse in a vertical direction; and
- the transparent lens body (60) extends from the emission side (61) against the direction of emission (A) to the rear side (62) and, in doing so, extends at least to a focal plane of the lens body (60).

2. A fixation target (70) according to claim 1, wherein the light generator (65) is configured as a passive light generator (65) that provides the electromagnetic radiation of the light field only when illuminated by the light source (71).

3. A fixation target (70) according to any one of the preceding claims, wherein the focal plane is arranged, at least in sections, exactly at the rear surface (62) of the lens body (60).

4. A fixation target (70) according to any one of claims 1 and/or 2 and according to claim 3, wherein the light generator (65) is provided by the rear surface (62) of the lens body (60) being blackened and/or roughened except for at least one section and/or area along the focal plane.

5. A fixation target (70) according to any one of claims 1 and 2, wherein the focal plane is located, at least in sections, inside the lens body.

6. A fixation target (70) according to any one of claims 1 and/or 2 and according to claim 5, wherein the light generator is provided by forming scattering centers inside the lens body along at least one section of the focal plane.

7. A fixation target (70) according to any one of the preceding claims, wherein at least one outer surface (61, 62, 63) of the lens body (60) is at least partially blackened and/or roughened.

8. A fixation target (70) according to any of the preceding claims, wherein at least one outer surface (61, 62, 63) of the lens body (60) is coated with an antireflective coating.

9. A fixation target (70) according to any one of the preceding claims, wherein the transparent lens body (60):
- is configured as a spherical or aspherical cylindrical lens with a focal line (69) located in the focal plane.

10. A fixation target (70) according to claims 1 and 9, wherein the light generator is configured as a light line generator (65) arranged approximately along at least a portion of the focal line (69) of the cylindrical lens.

11. A fixation target (70) according to any one of the preceding claims, wherein the transparent lens body (60) is molded and/or drawn from plastic.

12. A fixation target (70) according to any one of the preceding claims, wherein the fixation target (70) is configured such that
- the electromagnetic radiation of the light field is substantially diffuse in a first predeterminable plane, and
- the electromagnetic radiation of the light field is substantially parallel in a second predeterminable plane that is arranged approximately perpendicular to the first plane.

13. A centering device (10) for determining optical centering parameters and/or individual parameters of a spectacle wearer (30), comprising a fixation target (70) according to one of the preceding claims.

14. Use of a fixation target (70) according to any one of claims 1 through 12 as an aid for the defined alignment of the line of sight and/or at least one eye of a spectacle wearer (30), wherein a spatially extended light field is generated by means of the fixation target (70) and the spectacle wearer (30) looks at the light field.

15. A method for determining centering parameters and/or individual parameters of a spectacle wearer (30), wherein, by means of a fixation target (70) according to one of claims 1 through 12, the line of sight and/or at least one eye of the eyeglass wearer (30) is aligned in a defined manner, and the centering parameters and/or individual parameters are determined in this defined alignment position of the eyeglass wearer (30).

## Revendications

1. Cible de fixation (70) destinée à générer un champ lumineux pour orienter la direction du regard d'un porteur de lunettes (30) lors de la prise de mesures sur ce dernier (30) à l'aide d'un dispositif de centrage (10), comprenant :
- un corps de lentille transparent (60), au moyen duquel, en fonctionnement, un rayonnement électromagnétique du champ lumineux généré est mis en forme optiquement ;
- une face d'émission optique (61) du corps de lentille (60), à partir de laquelle, en fonctionnement, le rayonnement électromagnétique du champ lumineux généré sort dans une direction d'émission (A) ; et
- une face arrière (62) du corps de lentille (60), située sur une surface arrière du corps de lentille (60) opposée à la face de rayonnement (61) ;
dans laquelle :
- le long d'au moins une partie du plan focal, sur et/ou dans le corps de lentille (60), est formé un générateur de lumière (65) est formé, à partir duquel, en fonctionnement, un rayonnement électromagnétique est émis en direction du côté d'émission optique (61) de telle sorte que ce rayonnement électromagnétique, après être sorti du côté d'émission (61), forme le champ lumineux généré par la cible de fixation (70) ;
- la cible de fixation (70) comporte une source lumineuse (71) destinée à éclairer le générateur de lumière (65) de telle sorte que le générateur de lumière (65) forme un rayonnement électromagnétique émis par la source lumineuse (71) et l'émette sous forme de rayonnement électromagnétique en direction de la face d'émission optique (61) de telle sorte que ce rayonnement électromagnétique, après être sorti de la face d'émission (61), forme le champ lumineux généré par la cible de fixation de telle manière que le champ lumineux soit formé dans un plan horizontal parallèle à la direction de propagation et de manière diffuse dans une direction verticale ; et
- le corps de lentille transparent (60) s'étend depuis la face de sortie (61), dans le sens opposé à la direction de rayonnement (A), jusqu'à la face arrière (62) et, ce faisant, au moins jusqu'à un plan focal du corps de lentille (60).

2. Cible de fixation (70) selon la revendication 1, dans laquelle le générateur de lumière (65) est conçu comme un générateur de lumière passif (65) qui ne fournit le rayonnement électromagnétique du champ lumineux que lorsqu'il est éclairé par la source lumineuse (71).

3. Cible de fixation (70) selon l'une des revendications précédentes, dans laquelle le plan focal est disposé, au moins par sections, exactement au niveau de la face arrière (62) du corps de lentille (60).

4. Cible de fixation (70) selon l'une des revendications 1 et/ou 2 et selon la revendication 3, dans laquelle le générateur de lumière (65) est obtenu en rendant la face arrière (62) du corps de lentille (60) noircie et/ou rugueuse, à l'exception d'au moins une partie et/ou une zone située le long du plan focal.

5. Cible de fixation (70) selon l'une des revendications 1 et 2, dans laquelle le plan focal est disposé, au moins par sections, à l'intérieur du corps de lentille.

6. Cible de fixation (70) selon l'une des revendications 1 et/ou 2 et selon la revendication 5, dans laquelle le générateur de lumière est obtenu en formant, à l'intérieur du corps de lentille, le long d'au moins une partie du plan focal, des centres de diffusion.

7. Cible de fixation (70) selon l'une des revendications précédentes, dans laquelle au moins une surface extérieure (61, 62, 63) du corps de lentille (60) est au moins partiellement noircie et/ou rendue rugueuse.

8. Cible de fixation (70) selon l'une des revendications précédentes, dans laquelle au moins une surface extérieure (61, 62, 63) du corps de lentille (60) est recouverte d'un revêtement antireflet.

9. Cible de fixation (70) selon l'une des revendications précédentes, dans laquelle le corps de lentille transparent (60) :
- est réalisé sous la forme d'une lentille cylindrique sphérique ou asphérique présentant une ligne focale (69) qui est située dans le plan focal.

10. Cible de fixation (70) selon les revendications 1 et 9, dans laquelle le générateur de lumière est conçu comme un générateur de lignes lumineuses (65) qui est disposé approximativement le long d'au moins une partie de la ligne focale (69) de la lentille cylindrique.

11. Cible de fixation (70) selon l'une des revendications précédentes, dans laquelle le corps de lentille transparent (60) est moulé et/ou extrudé en matière plastique.

12. Cible de fixation (70) selon l'une des revendications précédentes, dans laquelle la cible de fixation (70) est conçue de telle sorte que
- le rayonnement électromagnétique du champ lumineux soit essentiellement diffus dans un premier plan prédéterminable et
- le rayonnement électromagnétique du champ lumineux soit essentiellement parallèle dans un deuxième plan prédéterminable, disposé approximativement perpendiculairement au premier plan.

13. Dispositif de centrage (10) destiné à déterminer des paramètres de centrage optiques et/ou des paramètres individuels d'un porteur de lunettes (30), comprenant une cible de fixation (70) selon l'une des revendications précédentes.

14. Utilisation d'une cible de fixation (70) selon l'une des revendications 1 à 12 comme aide à l'orientation définie de la direction du regard et/ou d'au moins un œil d'un porteur de lunettes (30), un champ lumineux étendu en surface étant généré au moyen de la cible de fixation (70) et le porteur de lunettes (30) regardant ce champ lumineux.

15. Procédé de détermination de paramètres de centrage et/ou de paramètres individuels d'un porteur de lunettes (30), dans lequel, à l'aide d'une cible de fixation (70) selon l'une des revendications 1 à 12, la direction du regard et/ou au moins un œil du porteur de lunettes (30) est orienté de manière définie, et les paramètres de centrage et/ou les paramètres individuels sont déterminés dans cette position définie du porteur de lunettes (30).
